# EUROPEAN PATENT APPLICATION

(11) **EP 1 837 017 A2**
(43) Date of publication of application: **26.09.2007**
(21) Application number: 07007764.9
(22) Date of filing: 22.01.1998
(51) Int. Cl.: A61K 9/16, A61K 48/00, C12N 15/87

(54) **Microparticles for delivery of nucleic acid**

(30) Priority: 22.01.1997 US 787547; 06.01.1998 US 3253
(62) Divisional of application: 98904700.6
(71) Applicant: MGI PHARMA Biologics, Inc., Cambridge, MA 02138 (US)
(72) Inventor: Hedley, Mary Lynne, Belmont, MA 02178 (US); Curley, Joanne. M., San Mateo, CA 94403 (US); Langer, Robert.S., Newton, MA 02158 (US); Lunsford,Lynn.B., Bedford, MA 01730 (US)
(74) Representative: Dzieglewska, Hanna Eva

(57) **Abstract**

Disclosed is a preparation of microparticles made up of a polymeric matrix and a nucleic acid expression vector. The polymeric matrix includes one or more synthetic polymers having a solubility in water of less than about 1 mg/1. At least 90% of the microparticles have a diameter less than about 100 microns. The nucleic acid is either RNA, at least 50% of which is in the form of closed circles, or circular DNA plasmid molecules, at least 50% of which are supercoiled.

## Description

### Background of the Invention

This invention relates to methods of delivering nucleic acids into cells.

Gene therapy is a highly promising technique for treatment of hereditary diseases, e.g., cystic fibrosis. Gene therapy can also be used when expression of gene products from genes which are not naturally found in the host cells is desired, for example, from genes encoding cytotoxic proteins targeted for expression in cancer cells.

Gene therapy can fall into several categories. It is sometimes desirable to replace a defective gene for the entire lifespan of a mammal, as in the case of an inherited disease such as cystic fibrosis, phenylketonuria, or severe combined immunodeficiency disease (SCID). In other cases, one may wish to treat a mammal with a gene that will express a therapeutic polypeptide for a limited amount of time, e.g., during an infection. Nucleic acids in the form of antisense oligonucleotides or ribozymes are also used therapeutically. Moreover, polypeptides encoded by nucleic acids can be effective stimulators of the immune response in mammals.

Various techniques have been used for introducing genes into cells, including infection with viral vectors, biolistic transfer, injection of "naked" DNA (US Patent No. 5,580,859), and delivery via liposomes or polymeric particles.

### Summary of the Invention

The invention is based on the discovery that microparticles containing nucleic acids having an appropriate size for phagocytosis can be made without adversely affecting nucleic acid integrity. These microparticles are highly effective vehicles for the delivery of polynucleotides into phagocytic cells.

In general, the invention features a preparation of microparticles (also called microspheres), each of which includes a polymeric matrix and a nucleic acid expression vector. The polymeric matrix includes one or more synthetic polymers having a solubility in water of less than about 1 mg/l; in the present context, synthetic is defined as non-naturally occurring. At least 90% of the microparticles have a diameter less than about 100 microns. The nucleic acid is either RNA, at least 50% (and preferably at least 70% or even 80%) of which is in the form of closed circles, or circular DNA plasmid molecules, at least 25% (and preferably at least 35%, 40%, 50%, 60%, 70%, or even 80%) of which are supercoiled. In some cases, it is desirable for at least 90% of the microparticles to have a diameter less than about 20 microns, and preferably less than about 11 microns. The nucleic acid can be either distributed throughout the microparticle or can be in the core of a hollow core microparticle.

The preparation can also include a stabilizer compound (e.g., a carbohydrate, a cationic compound, or a DNA-coridensing agent). A stabilizer compound is a compound that acts to protect the nucleic acid (e.g., to keep it supercoiled) at any time during the production of microparticles. Examples of stabilizer compounds include dextrose, sucrose, dextran, polyvinyl alcohol, cyclodextrin, dextran sulfate, cationic peptides, and lipids such as hexadecyltrimethylammonium bromide. The stabilizer compound can remain associated with the DNA after a later release from the polymeric matrix.

Another embodiment of the invention features a microparticle less than about 20 microns in diameter, including a polymeric matrix and nucleic acid. The polymeric matrix is made from one or more synthetic polymers having a solubility in water of less than about 1 mg/l. At least 50% (and preferably at least 70% or even 80%) of the nucleic acid molecules are in the form of supercoiled DNA.

The polymeric matrix can be biodegradable. Biodegradable is used here to mean that the polymers degrade over time into compounds which are known to be cleared from the host cells by normal metabolic pathways. Generally, a biodegradable polymer will be substantially metabolized within about 1 month after injection into a patient, and certainly within about 2 years. In certain cases, the polymeric matrix can be made of a single synthetic, biodegradable copolymer, e.g., poly-lactic-co-glycolic acid (PLGA). The ratio of lactic acid to glycolic acid in the copolymer can be within the range of about 1:2 to about 4:1 by weight, preferably within the range of about 1:1 to about 2:1 by weight, and most preferably about 65:35 by weight. In some cases, the polymeric matrix also includes a targeting molecule such as a ligand, receptor, or antibody, to increase the specificity of the microparticle for a given cell type or tissue type.

For certain applications, the microparticle has a diameter of less than about 11 microns. The microparticle can be suspended in an aqueous solution (e.g., for delivery by injection) or can be in the form of a dry solid (e.g., for storage or for delivery via inhalation or implantation) . The nucleic acid can be an expression control sequence operatively linked to a coding sequence. Expression control sequences include, for example, any nucleic acid sequences known to regulate transcription or translation, such as promoters, enhancers, or silencers. In preferred examples, at least 60% or 70% of the DNA is supercoiled. More preferably, at least 80% is supercoiled.

In another embodiment, the invention features a microparticle less than about 20 microns in diameter, including a polymeric matrix and a nucleic acid molecule (preferably in closed, circular form), wherein the nucleic acid molecule includes an expression control sequence operatively linked to a coding sequence. The expression product encoded by the coding sequence can be a polypeptide at least 7 amino acids in length, having a sequence essentially identical to the sequence of either a fragment of a naturally-occurring mammalian protein or a fragment of a naturally-occurring protein from an agent which infects or otherwise harms a mammal; or a peptide having a length and sequence which permit it to bind to an MHC class I or II molecule. Examples are set forth in WO 94/04171, hereby incorporated by reference.

*Essentially identical* in the context of a DNA or polypeptide sequence is defined here to mean differing no more than 25% from the naturally occurring sequence, when the closest possible alignment is made with the reference sequence and where the differences do not adversely affect the desired function of the DNA or polypeptide in the methods of the invention. The phrase fragment of a protein is used to denote anything less than the whole protein.

The peptide or polypeptide can be linked to a trafficking sequence. The term "trafficking sequence" refers to an amino acid sequence which causes a polypeptide to which it is fused to be either secreted from the cell or transported to a specific compartment of the cell, e.g., the nucleus, endoplasmic reticulum, a lysosome, or an endosome.

In the embodiment where the expression product includes a peptide having a length and sequence which permit it to bind an MHC class I or II molecule, the expression product is typically immunogenic. The expression product can have an amino acid sequence that differs from the sequence of a naturally occurring protein recognized by a T cell in the identity of not more than 25% of its amino acid residues, provided that it can still be recognized by the same T cell and can alter the cytokine profile of the T cell (i.e., an "altered peptide ligand"). The differences between the expression product and the naturally occurring protein can, for example, be engineered to increase cross-reactivity to pathogenic viral strains or HLA-allotype binding.

Examples of expression products include amino acid sequences at least 50% identical to the sequence of a fragment of myelin basic protein (MBP), proteolipid protein (PLP), invariant chain, GAD65, islet cell antigen, desmoglein, α-crystallin, or β-crystallin, where the fragment can bind the MHC class II molecule. Table 1 lists many of such expression products that are thought to be involved in autoimmune disease. Fragments of these proteins can be essentially identical to any one of SEQ ID NOS: 1-46 such as MBP residues 80-102 (SEQ ID NO: 1), PLP residues 170-191 (SEQ ID NO: 2), or invariant chain residues 80-124 (SEQ ID NO: 3). Other fragments are listed in Table 2.

Alternatively, the expression product can include an amino acid sequence essentially identical to the sequence of an antigenic portion of any of the tumor antigens listed in Table 3 such as those encoded by the human papillomavirus E6 and E7 genes, Her2/neu gene, the prostate specific antigen gene, the melanoma antigen recognized by T cells (MART) gene, or the melanoma antigen gene (MAGE). Again, the expression product can be engineered to increase cross-reactivity.

In still other cases, the expression product includes an amino acid sequence essentially identical to the sequence of an antigenic fragment of a protein naturally expressed by a virus, e.g., a virus which chronically infects cells, such as human papillomavirus (HPV), human immunodeficiency virus (HIV), herpes simplex virus (HSV), hepatitis B virus (HBV), or hepatitis C virus (HCV); a bacterium, such as mycobacteria; or a parasitic eukaryote, such as *Plasmodium* species. A representative list of such class I-binding fragments as well as fragments of tumor antigens is included in Table 4.

In another embodiment, the invention features a microparticle less than about 20 microns in diameter, including a polymeric matrix and a nucleic acid molecule, wherein the nucleic acid molecule includes an expression control sequence operatively linked to a coding sequence. The expression product encoded by the coding sequence is a protein which, when expressed, downregulates an immune response. Examples of such proteins include tolerizing proteins, MHC blocking peptides, receptors, transcription factors, and cytokines.

In another embodiment, the invention features a process for preparing microparticles. A first solution, including a polymer dissolved in an organic solvent, is mixed (e.g., with sonication, with vortexing, or in a microfluidizer) with a second solution, which includes a nucleic acid dissolved or suspended in a polar or hydrophilic solvent (e.g., an aqueous buffer solution containing, for instance, ethylenediaminetetraacetic

**TABLE 1: Autoantigens**

| **Disease** | **Associated Antigen** | **Notes** |
|---|---|---|
| Coeliac disease | α-Gliadin | a |
| Goodpasture's syndrome | Basement membrane collagen | a |
| Craves' disease | Thyroid stimulating Hormone (TSH) receptor | a |
| Hashimoto's disease | Thyroglobulin | a |
| Isaac's syndrome | voltage-gated potassium channels | b |
| Insulin-dependent diabatee | Glutamic acid decarboxylase (GAD) | a |
| | Insulin receptor | a |
| | Insulin associated antigen (IA-w) | a |
| | Hsp | b |
| Lambert-Eaton myasthenic syndrome (LEMS) | Synaptogamin in voltage-gated calcium channels | b |
| Multiple sclerosis | Myelin basic protein (MBP) | a |
| | Proteolipid protein (PLP) Myelin oligodeadrocyte-associated | a |
| | protein (MOG) | a |
| | αB-crystallin | a |
| Myasthenia Gravis | Acetyl choline receptor | a |
| Paraneoplastic encephalitis | RNA-binding protein HuD | b |
| Pemphigus vulgaris | "PeV antigen complex" | a |
| | Desmoglein (DG) | c |
| Primary Biliary cirrhosis | Dihydrolipoamide acetyltransferase | b |
| | Pyruvate dehydrogenase complex 2 (PDC-E2) | d |
| Progressive systemic | DNA topoisomerase | a |
| sclerosis | RNA polymerase | a |
| Rheumatoid arthritis | Immunoglobulin Fc Collagen | a |
| Scleroderma | Topoisomerase I | b |
| Stiff-man syndrome | Glutamic acid decarboxylase (GAD) | a |
| systemic lupus erythematosus | ds-DNA | a |
| Uvsitis | Interphotoreceptor retinoid-binding protein | b |
| | S antigen (rod out segment) | b |

| | | |
|---|---|---|
| References: a) HLA and Autoimmune Disease, R. Heard, pg. 123-151 in HLA & Disease, Academic Press, Now York, 1994, (R. Lechler, ed.) b) Call 80, 7-10 (1995) c) Cell 67, 669-877 (1991) d) JEM 181, 1835-1845 (1995) | | |

**TABLE 2: Class II Associated Peptides**

| **Peptide** | **SEQ ID NO:** | **Source Protein** |
|---|---|---|
| GRTQDENPVVHFFKNIVTPRTPP | 1 | MBP 80-102 |
| AVYVYIYFNTWTTCQFIAPPFK | 2 | PLP 170-191 |
| FKMRMATPLLMQA TVGLQLIQLINVDEVNQIV | 3 | Invariant chain 88-100 |
| TTNVRLKQQWVDYNLKW | 4 | AChR α 32-67 |
| QIVTTNVRLKQQWVDYNLKW | 5 | AChR α 48-67 |
| QWVDYNL | 6 | AChR α 59-65 |
| GGVKKIHIPSEKIWRPDL | 7 | AChR α 73-90 |
| AIVKFTXVLLQY | 8 | AChR α 101-112 |
| WTPPAIFKSYCEIIVTEFPF | 9 | AChR α 118-137 |
| MKLGTWTYDGSVV | 10 | AChR α 144-156 |
| MKLGIWTYDGSVV | 11 | AChR a 144-157 analog(I-148) |
| WTYDGSVVA | 12 | AChR α 149-157 |
| SCCPDTPYLDITYHFVM | 13 | AChR α 191-207 |
| DTPYLDITYHFVMQRLPL | 14 | AChR α 195-212 |
| FIVNVIIPCLLFSFLTGLVFY | 15 | AChR a 214-234 |
| LLVIVELIPSTSS | 16 | AChR α 257-269 |
| STHVMPNWVRKVFIDTIPN | 17 | AChR α 304-322 |
| NWVRKVFIDTIPNIMFFS | 18 | AChR α 310-327 |
| IPNIMFFSTMKRPSREKQ | 19 | AChR α 320-337 |
| AAAEWKYVAMVMDHIL | 20 | AChR α 395-410 |
| IIGTLAVFAGRLIELNQQG | 21 | AChR α 419-437 |
| GQTIEWIFIDPEAFTENGEW | 22 | AChR γ 165-184 |
| MAHYNRVPALPFPGDPRPYL | 23 | AChR γ 476-495 |
| LNSKIAFKIVSQEPA | 24 | desmoglein 3 190-204 |
| TPMFLLSRNTGEVRT | 25 | desmoglein 3 206-220 |
| PLGFFPDHQLDPAFGA | 26 | HBS preS1 10-25 |
| LGFFPDHQLDPAFGANS | 27 | HBS preS1 11-27 |
| FFLLTRILTI | 28 | HBS Ag 19-28 |
| RILTIPQSLD | 29 | HBS Ag 24-33 |
| TPTLVEVSRNLGK | 30 | HSA 444-456 |
| AKTIAYDEEARR | 31 | hsp 65 2-13 |
| VVTVRAERPG | 32 | hsp 18 61-70 |
| SQRHGSKYLATASTMDHARHG | 33 | MBP 7-27 |
| RDTGILDSIGRFFGGDRGAP | 34 | MBP 33-52 |
| QKSHGRTQDENPVVHFFKNI | 35 | MBP 74-93 |
| DENPVVHFFKNIVT | 36 | KBP 84-97 |
| ENPVVHFFKNIVTPR | 37 | MBP 85-99 |
| HPFKNIVTPRTPP | 38 | MBP 90-102 |
| KGFKGVDAQSTLSK | 39 | MBP 139-152 |
| VDAQGTLSKIFKLGGRDSRS | 40 | MBP 144-163 |
| LMQYIDANSKFIGITELKK | 41 | Tetanus Toxoid 828-846 |
| QYIKANSKFIGIT | 42 | Tetanus Toxoid 830-842 |
| FNNFTVSFWLRVPK | 43 | Tetanus Toxoid 947-960 |
| SFWLRVPKVSASHLE | 44 | Tetanus Toxoid 953-967 |
| KFIIKRYTPNNEIDSF | 45 | Tetanus Toxoid 1174-1189 |
| GQIGNDPNRDIL | 46 | Tetanus Toxoid 1273-1284 |

**TABLE 3: Tumor Antigens**

| **Cancer** | **Associated Antigen** |
|---|---|
| Melanoma | BAGE 2-10 |
| Breaet/Ovarian | c-ERB2 (Her2/neu) |
| Burkitt's lymphoma/Bodgkin's lymphoma | EBNA-1 |
| Burkitt's lymphoma/Hodgkin's lymphoma | EBNA-2 |
| Burkitt's lymphoma/Hodgkin's lymphoma | EBNA-3 |
| Burkitt's lymphoma/Hodgkin's lymphoma | EBNA-3A |
| Burkitt's lymphoma/Hodgkin's lymphoma | EBNA-3C |
| Burkitt's lymphoma/Hodgkin's lymphoma | EBNA-4 |
| Burkitt's lymphoma/Hodgkin's lymphoma | EBNA-6 |
| Burkitt's lymphoma/Hodgkin's lymphoma | EBV |
| Burkitt's lymphoma/Hodgkin's lymphoma | EBV LMP2A |
| Melanoma | GAGE-1 |
| Melanoma | gp75 |
| Cervical | HPV 16 E6 |
| cervical | HPV 16 E7 |
| Cervical | HPV 18 E6 |
| Cervical | HPV 1B E7 |
| Melanoma | MAG |
| Melanoma | MAGE-1 |
| Melanoma | MAGE-2 |
| Melanoma | MAGE-3 |
| Melanoma | MAGE-4b |
| Melanoma | MAGE-5 |
| Melanoma | MAGE-6 |
| Melanoma | MART-1/Melan-A |
| Pancreatic/Breast/ovarian | MUC-1 |
| Melanoma | HUM-1-B |
| Breast/Colorectal/Burkitt's lymphoma | p53 |
| Melanoma | Pmel 17(gp100) |
| Prostate | PSA Prostate Specific Antigen |
| Melanoma | Tyrosinase |
| | CEA Curcinoembryonic Antigen |
| | LRP Lung Resistance Protein |
| | Bc1-2 |
| | Ki-67 |

**TABLE 4: Class I associated tumor and pathogen peptides**

| **Peptide** | **SEQ ID NO:** | **Source Protein** |
|---|---|---|
| AAPAVFLAL | 47 | BAGE 2-10 |
| YRPRPRRY | 48 | GAGE-1 9-16 |
| EADPTGHSY | 49 | MAGE-1 161-169 |
| SAYGEPRKL | 50 | MAGE-1 230-238 |
| EVDPIGHLY | 51 | MAGE-3 161-169 |
| FLWGPRALV | 52 | MAGE-3 271-279 |
| GIGILTV | 53 | MART-1 29-35 |
| ILTVILGV | 54 | MART-1 32-39 |
| STAPPAHGV | 55 | MUC-1 9-17 |
| EEKLIVVLF | 56 | MUM-1 261-269 |
| MLLAVLYCL | 57 | TYROSINASE 1-9 |
| SEIWRDIDF | 58 | TYROSINASE 192-200 |
| AFLPWHRLF | 59 | TYROSINASE 206-214 |
| YMNGTMSQV | 60 | TYROSINASE 369-376 |
| KTWGQYWQV | 61 | PMEL 17 (GP100) 154-162 |
| ITDQVPFSV | 62 | PMEL 17 (GP100) 209-217 |
| YLEPGPTVA | 63 | PMEL 17 (GP100) 280-288 |
| LLDGTATLRL | 64 | PMEL 17 (GP100) 476-485 |
| ELNEALELEK | 65 | p53 343-351 |
| STPPPGTRV | 66 | p53 149-157 |
| LLPENNVLSPL | 67 | p53 25-35 |
| LLGRNSFEV | 68 | p53 264-272 |
| RHPEAAPPV | 69 | p53 65-73 |
| RIPGSLAFL | 70 | HER-2/neu 369-377 |
| IISAVVGIL | 71 | HER-2/neu 654-662 |
| CLTSTVQLV | 72 | HER-2/neu 789-797 |
| YLEDVRLV | 73 | HER-2/neu 835-842 |
| VLVKSPNHV | 74 | HER-2/neu 851-859 |
| RFRELVSEFSRM | 75 | HER-2/neu 968-979 |
| LLRLSEPAEL | 76 | PSA 119-128 |
| DLPTQEPAL | 77 | PSA 136-144 |
| KLQCVD | 78 | PSA 166-171 |
| VLVASRGRAV | 79 | PSA 36-45 |
| VLVBPQWVL | 80 | PSA 49-57 |
| DMSLLKNRFL | 81 | PSA 98-107 |
| QWHSTMBQ | 82 | HBV envelope 121-130 |
| VLQAGFF | 83 | HBV envelope 177-184 |
| LLLCLIFL | 84 | HBV envelope 250-257 |
| LLDYQGML | 85 | HBV envelope 260-267 |
| LLVPFV | 86 | HBV envelope 338-343 |
| SILSPFMPLL | 87 | HBV envelope 370-379 |
| PLLPIFFCL | 88 | HBV envelope 377-385 |
| ILSTLPETTV | 89 | HBV core 529-538 |
| FLPSDFFPSV | 90 | HBV core 47-56 |
| KLHLYSHPI | 91 | HBV polymerase 489-498 |
| ALMPLYACI | 92 | HBV polymerase 642-651 |
| HLYSHPIIL | 93 | HBV polym. 1076-1084 |
| FLLSLGIHL | 94 | HBV polym. 1147-1153 |
| HLLVGSSGL | 95 | HBV polymerase 43-51 |
| GLSRYVARL | 96 | HBV polymerase 455-463 |
| LLAQFTSAI | 97 | HBV polymerase 527-535 |
| YMDDVVLGA | 98 | HBV polymerase 551-559 |
| GLYSSTVPV | 99 | HBV polymerase 61-69 |
| NLSWL | 100 | HBV polymerase 996-1000 |
| KLPQLCTEL | 101 | HPV 16 E6 18-26 |
| LQTTIHDII | 102 | HPV 16 E6 26-34 |
| FAFRDLCIV | 103 | HPV 16 E6 52-60 |
| YMLDLQPET | 104 | HPV 16 E7 11-19 |
| TLHEYMLDL | 105 | HPV 16 E7 7-15 |
| LLHGTLGIV | 106 | HPV 16 E7 82-90 |
| TLGIVCPI | 107 | HPV 16 E7 86-93 |
| LLMGTLGIVCPI | 108 | HPV 16 E7 82-93 |

acid, or tris(hydroxymethyl)amitidmethane, or combinations thereof, optionally including a DNA condensing agent such as a cationic peptide, a lipid, or a dendrimer). The mixture forms a first emulsion. The first emulsion is then mixed with a third solution which includes an organic compound (e.g., polyvinyl alcohol), to form a second emulsion containing microparticles of polymer matrix and nucleic acid. The mixing steps can be executed, for example, in a homogenizer, vortex mixer, microfluidizer, or sonicator. Both mixing steps are carried out in a manner that minimizes shearing of the nucleic acid while producing microparticles on average smaller than 100 microns in diameter.

The second solution can, for example, be prepared by column chromatography or otherwise purifying the nucleic acid (e.g., by ethanol or isopropanol precipitation), then suspending the purified or precipitated nucleic acid in an aqueous, polar, or hydrophilic solution.

The second solution can optionally include a surfactant, a DNA-condensing agent, or a stabilizer compound (e.g., 1-10% dextrose, sucrose, dextran, or other carbohydrates, polyvinyl alcohol, cyclodextrin, hexadecyltrimethylammonium bromide, or dextran sulfate) that can stabilize the nucleic acid or emulsion by keeping the nucleic acid supercoiled during encapsulation or throughout of microparticle formation.

The second emulsion is optionally mixed with a fourth solution including an organic compound such as polyvinyl alcohol. The second emulsion can optionally be exposed (i.e., alone or as a mixture with the fourth emulsion) to an elevated temperature (e.g., room temperature to about 60°C), for example, to facilitate more rapid evaporation of the solvents.

The procedure can include the additional step of washing the microparticles with an aqueous solution to remove organic compounds, thereby producing washed microparticles. The washed microparticles can then be subjected to a temperature below 0°C, to produce frozen microparticles, which are in turn lyophilized to produce lyophilized microparticles. The microparticles can optionally be suspended in an excipient, such as Tween-80, mannitol, sorbitol, or carboxymethyl-cellulose, prior to or after lyophilization (if any).

When desired, the procedure can include the additional step of screening the microparticles to remove those larger than 100 microns (or even 20 microns) in diameter.

Still another embodiment of the invention features a preparation of microparticles which include a polymeric matrix, a proteinaceous antigenic determinant, and a DNA molecule which encodes an antigenic polypeptide that can be different from, or the same as, the aforementioned proteinaceous antigen determinant. The antigenic determinant contains an epitope which can elicit an antibody response. The antigenic polypeptide expressed from the DNA can induce a T cell response (e.g., a CTL response). The DNA can be plasmid DNA, and can be combined in the same microparticle as the antigenic determinant, or the two can be in distinct microparticles which are then mixed together. In some cases, an oligonucleotide, rather than a proteinaceous antigenic determinant, can be encapsulated together with a nucleic acid plasmid. The oligonucleotide can have antisense or ribozyme activity, for example.

In another embodiment, the invention features a method of administering nucleic acid to an animal by introducing into the animal (e.g., a mammal such as a human, non-human primate, horse, cow, pig, sheep, goat, dog, cat, mouse, rat, guinea, hamster, or ferret) any of the microparticles described in the paragraphs above. The microparticles can be provided suspended in a solution (e.g., an aqueous solution) or any other suitable formulation, and can be, for example, injected or implanted (e.g., surgically) into the animal, or administered by inhalation (e.g., intranasally). They can optionally be delivered in conjunction with a protein such as a cytokine, a hormone, an interferon, or an antigen.

In another embodiment, the invention features a preparation of microparticles, each of which includes a polymeric matrix, a stabilizing compound, and a nucleic acid expression vector. The polymeric matrix includes one or more synthetic polymers having a solubility in water of less than about 1 mg/l; in the present context, synthetic is defined as non-naturally occurring. At least 90% of the microparticles have a diameter less than about 100 microns. The nucleic acid is either RNA, at least 50% (and preferably at least 70% or even 80%) of which is in the form of closed circles.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, the preferred methods and materials are described below. All publications, patent applications, patents, and other references mentioned herein are incorporated by reference in their entirety. In case of conflict, the present application, including definitions, will control. In addition, the materials, methods, and examples are illustrative only and not intended to be limiting.

Other features and advantages of the invention will be apparent from the following detailed description, and from the claims.

### Brief Description of the Drawings

Figs. 1A to 1C are a set of three plasmid maps, of the pvA2.1/4, luciferase, and VSV-Npep plasmids, respectively.
Fig. 2 is a plot of size distribution of DNA-containing microparticles as analyzed on a COULTER™ counter.
Figs. 3A and 3B are a set of photographs of two agarose electrophoresis gels indicating degree of DNA supercoiling as a function of different homogenization speeds and durations.
Figs. 4A and 4B are a pair of FACS printouts comparing cell populations in the absence or presence of microparticles.
Figs. 5 to 9 are plots of specific lysis versus effector:target ratio.

### Description of the Preferred Embodiments

The microparticles of the invention are formulated in one of two ways: (1) to maximize delivery into the patient's phagocytic cells, or (2) to form a deposit in the tissues of the patient, from which the nucleic acid is released gradually over time; upon release from the microparticle, the nucleic acid is taken up by neighboring cells (including antigen presenting cells, or APCs). In both cases, maintaining the integrity of the DNA is a priority. For plasmid DNA, this means maximizing the percentage of plasmid molecules that are supercoiled and thus more stable or more capable of more efficient transfection or expression than non-supercoiled (i.e., nicked or linear) plasmids. Means for protecting the integrity of the nucleic acid include minimizing the shearing forces to which the nucleic acid is necessarily exposed in the process of microparticle formation, limiting sonication and mixing times during preparation, and adding buffers or other stabilizer compounds during nucleic acid isolation and encapsulation. For example, it is necessary to achieve a balance between sonication time and intensity. These techniques are discussed below.

The microparticles of the invention can be used in the manufacture of a medicament for the treatment of, for example, cancer, any of the autoimmune diseases listed in Table 1, or any other condition treatable with a particular defined nucleic acid.

Phagocytosis of microparticles by macrophages, dendritic cells, and other APCs is an effective means for introducing the nucleic acid into these cells. Phagocytosis by these cells can be increased by maintaining a particle size below about 20 µm, and preferably below about 11 µm. The type of polymer used in the microparticle can also affect the efficiency of uptake by phagocytic cells, as discussed below.

The microparticles can be delivered directly into the bloodstream (i.e., by intravenous or intraarterial injection or infusion) where uptake by the phagocytic cells of the reticuloendothelial system (RES) is desired. Alternatively, one can target, via subcutaneous injection, take-up by the phagocytic cells of the draining lymph nodes. The microparticles can also be introduced intradermally (i.e., to the APCs of the skin, such as dendritic cells and Langerhans cells), intramuscularly, or intranasally or via other mucosal sites (i.e., for mucosal immunity). Finally, the microparticles can be introduced into the lung (e.g., by inhalation of powdered microparticles or of a nebulized or aerosolized solution containing the microparticles), where the particles are picked up by the alveolar macrophages.

Once a phagocytic cell phagocytoses the microparticle, the nucleic acid is released into the interior of the cell. Upon release, it can perform its intended function: for example, expression by normal cellular transcription/translation machinery (for an expression vector), or alteration of cellular processes (for antisense or ribozyme molecules).

Because these microparticles are passively targeted to macrophages and other types of phagocytic cells, they represent a means for modulating immune function. Macrophages and dendritic cells serve as professional APCs, expressing both MHC class I and class II molecules. Delivery, via microparticles, of an expression vector encoding a foreign antigen which binds to an MHC class I or class II molecule will induce a host T cell response against the antigen, thereby conferring host immunity.

Where the expression vector encodes a blocking peptide (See, e.g., WO 94/04171) that binds to an MHC class II molecule involved in autoimmunity, presentation of the autoimmune disease-associated self peptide by the class II molecule is prevented, and the symptoms of the autoimmune disease alleviated.

In another example, an MHC binding peptide that is identical or almost identical to an autoimmunity-inducing peptide can affect T cell function by tolerizing or anergizing the T cell. Alternatively, the peptide could be designed to modulate T cell function by altering cytokine secretion profiles following recognition of the MHC/peptide complex. Peptides recognized by T cells can induce secretion of cytokines that cause B cells to produce antibodies of a particular class, induce inflammation, and further promote host T cell responses.

Induction of immune responses can require several factors. It is this multifactorial nature that provides impetus for attempts to manipulate immune related cells on multiple fronts, using the microparticles of the invention. For example, microparticles can be prepared which carry both DNA and polypeptides within each microparticle. These dual-function microparticles are discussed below.

### CTL Responses

Class I molecules present antigenic peptides to immature T cells. To fully activate T cells, factors other than the antigenic peptide are required. Non-specific proteins such as interleukin-2 (IL-2), IL-12, and gamma interferon (γ-IFN) promote CTL responses and can be provided together with DNA encoding polypeptides which include CTL epitopes. Alternatively, proteins which bear helper T (T_{H}) determinants can be included with DNA encoding the CTL epitope. T_{H} epitopes promote secretion of cytokines from T_{H} cells and play a role in the differentiation of nascent T cells into CTLs.

Alternatively, proteins or nucleic acids which promote migration of lymphocytes and macrophages to a particular area could be included in microparticles along with appropriate DNA molecules. Uptake of the DNA is enhanced as a result, because release of the protein would cause an influx of phagocytic cells and T cells as the microparticle degrades. The macrophages would phagocytose the remaining microparticles and act as APC, and the T cells would become effector cells.

### Antibody Responses

Elimination of certain infectious agents from the host may require both antibody and CTL responses. For example, when the influenza virus enters a host, antibodies can often prevent it from infecting host cells. However, if cells are infected, then a CTL response is required to eliminate the infected cells and to prevent the continued production of virus within the host.

In general, antibody responses are directed against conformational determinants and thus require the presence of a protein or a protein fragment containing such a determinant. In contrast, T cell epitopes are linear determinants, typically just 7-25 residues in length. Thus, when there is a need to induce both a CTL and an antibody response, the microparticles can include both an antigenic protein and the DNA encoding a T cell epitope.

Slow release of the protein from microparticles would lead to B cell recognition and subsequent secretion of antibody. In addition, phagocytosis of the microparticles would cause APCs (1) to express the DNA of interest, thereby generating a T cell response; and (2) to digest the protein released from the microparticles, thereby generating peptides which are subsequently presented by class I or class II molecules, or both. Presentation by class II molecules promotes both antibody and CTL responses, since T_{H} cells activated by the class II/peptide complexes would secrete non-specific cytokines.

### Immunosuppression

Certain immune responses lead to allergy and autoimmunity, and so can be deleterious to the host. In these instances, there is a need to inactivate tissue-damaging immune cells. Immunosuppression can be achieved with microparticles bearing DNA that encodes altered peptide ligands, tolerizing peptides, or epitopes that down-regulate T_{H} cells and CTLs (e.g., "blocking" peptides). In these microparticles, the effect of the immunosuppressive DNA could be amplified by including certain proteins in the carrier microparticles with the DNA. A list of such proteins includes antibodies, receptors, transcription factors, hormones, and the interleukins.

For example, antibodies to stimulatory cytokines, cell surface receptors, or homing proteins, such as integrins or intercellular adhesion molecules (ICAMs), can increase the efficacy of the immunosuppressive DNA epitope. These proteins serve to inhibit the responses of already-activated T cells, while the DNA further prevents activation of nascent T cells. Induction of T cell regulatory responses can be influenced by the cytokine milieu present when the T cell receptor (TCR) is engaged. Cytokines such as IL-4, IL-10, and IL-6 promote T_{H}2 differentiation in response to the DNA-encoded epitope. T_{H}2 responses can inhibit the formation of T_{H}1 cells and the corresponding deleterious responses which result in the pathologies of rheumatoid arthritis, multiple sclerosis and juvenile diabetes.

Inclusion of proteins comprising soluble forms of costimulatory molecules (e.g., CD-40, gp-39, B7-1, and B7-2) or molecules involved in apoptosis (e.g., Fas, FasL, Bcl2, caspase, bax, TNFα, TNF receptors) is another way to inhibit activation of particular T cell and/or B cells responses. For example, B7-1 is involved in the activation of T_{H}1 cells, and B7-2 activates T_{H}2 cells. Depending on the response that is required, one or the other of these proteins could be included in the microparticle with the DNA, or could be supplied in separate microparticles mixed with the DNA-containing microparticles.

### Microparticles for Implantation

A second microparticle formulation of the invention is intended not to be taken up directly by cells, but rather to serve primarily as a slow-release reservoir of nucleic acid that is taken up by cells only upon release from the microparticle through biodegradation. The nucleic acid can be complexed to a stabilizer (e.g., to maintain the integrity of the nucleic acid during the slow-release process). The polymeric particles in this embodiment should therefore be large enough to preclude phagocytosis (i.e., larger than 5 µm and preferably larger than 19 µm). Such particles are produced by the methods described above for making the smaller particles, but with less vigorous mixing of the aforementioned first or second emulsions. That is to say, a lower homogenization speed, vortex mixing speed, or sonication setting can be used to obtain particles having a diameter around 100 µm rather than 5 µm. The time of mixing, the viscosity of the first emulsion, or the concentration of polymer in the first solution can also be altered to affect particle dimension.

The larger microparticles can be formulated as a suspension, a powder, or an implantable solid, to be delivered by intramuscular, subcutaneous, intradermal, intravenous, or intraperitoneal injection; via inhalation (intranasal or intrapulmonary); orally; or by implantation. These particles are useful for delivery of any expression vector or other nucleic acid for which slow release over a relatively long term is desired: e.g., an antisense molecule, a gene replacement therapeutic, a means of delivering cytokine-based, antigen-based, or hormone-based therapeutic, or an immunosuppressive agent. The rate of degradation, and consequently of release, varies with the polymeric formulation. This parameter can be used to control immune function. For example, one would want a relatively slow release for delivery of IL-4 or IL-10, and a relatively rapid release for delivery of IL-2 or γ-IFN.

### Composition of Polymeric Particles

Polymeric material is obtained from commercial sources or can be prepared by known methods. For example, polymers of lactic and glycolic acid can be generated as described in US Patent No. 4,293,539 or purchased from Aldrich.

Alternatively, or in addition, the polymeric matrix can include polylactide, polyglycolide, poly(lactide-co-glycolide), polyanhydride, polyorthoester, polycaprolactone, polyphosphazene, proteinaceous polymer, polypeptide, polyester, or polyorthoester. ,

Preferred controlled release substances which are useful in the formulations of the invention include the polyanhydrides, co-polymers of lactic acid and glycolic acid wherein the weight ratio of lactic acid to glycolic acid is no more than 4:1, and polyorthoesters containing a degradation-enhancing catalyst, such as an anhydride, e.g., 1% maleic anhydride. Since polylactic acid takes at least one year to degrade *in vivo,* this polymer should be utilized by itself only in circumstances where extended degradation is desirable.

### Association of Nucleic Acid and Polymeric Particles

Polymeric particles containing nucleic acids can be made using a double emulsion technique. First, the polymer is dissolved in an organic solvent. A preferred polymer is polylactic-*co*-glycolic acid (PLGA), with a lactic/glycolic acid weight ratio of 65:35, 50:50, or 75:25. Next, a sample of nucleic acid suspended in aqueous solution is added to the polymer solution and the two solutions are mixed to form a first emulsion. The solutions can be mixed by vortexing or shaking, or the mixture can be sonicated. The emulsion can be made in a microfluidizer. Most preferable is any method by which the nucleic acid receives the least amount of damage in the form of nicking, shearing, or degradation, while still allowing the formation of an appropriate emulsion. For example, acceptable results can be obtained with a Vibra-cell model VC-250 sonicator with a 1/8" microtip probe, at setting #3, or by controlling the pressure in the microfluidizer.

During this process, water droplets (containing the nucleic acid) form within the organic solvent. If desired, one can isolate a small amount of the nucleic acid at this point in order to assess integrity, e.g., by gel electrophoresis.

It is found that alcohol precipitation, or other modes of purification, of the nucleic acid prior to suspension in the aqueous solution can improve encapsulation efficiency. Precipitation with ethanol resulted in up to a 147% increase in incorporated DNA and precipitation with isopropanol increased incorporation by up to 170%.

The nature of the aqueous solution can affect the yield of supercoiled DNA. For example, the presence of detergents such as polymyxin B, which are often used to remove endotoxins during the preparation and purification of DNA samples, can lead to a decrease in DNA encapsulation efficiency. It may be necessary to balance the negative effects on encapsulation efficiency with the positive effects on supercoiling, especially when detergents, surfactants, and/or stabilizers are used during encapsulation. Furthermore, addition of buffer solutions containing either tris(hydroxymethyl)aminomethane (TRIS), ethylenediaminetetraacetic acid (EDTA), or a combination of TRIS and EDTA (TE) resulted in stabilization of supercoiled plasmid DNA, according to analysis by gel electrophoresis. pH effects are also observed. Other stabilizing compounds, such as dextran sulfate, dextrose, dextran, CTAB, cyclodextrin, and sucrose, were also found to enhance the stability and degree of supercoiling of the DNA, either alone or in combination with the TE buffer. Combinations of stabilizers can be used to increase the amount of encapsulated, supercoiled DNA. Stabilizers such as charged lipids (e.g., CTAB), cationic peptides, or dendrimers (J. Controlled Release, 39:357, 1996) can condense or precipitate the DNA. Moreover, stabilizers can have an effect on the physical nature of the particles formed during the encapsulation procedure. For example, the presence of sugars or surfactants during the encapsulation procedure can generate porous particles with porous interior or exterior structures, allowing for a more rapid exit of a drug from the particle. The stabilizers can act at any time during the preparation of the microspheres: during encapsulation or lyophilization, or both, for example.

The first emulsion is then added to an organic solution, allowing formation of microparticles. The solution can be comprised of, for example, methylene chloride, ethyl acetate, or acetone, preferably containing polyvinyl alcohol (PVA), and most preferably having a 1:100 ratio of the weight of PVA to the volume of the solution. The first emulsion is generally added to the organic solution with stirring in a homogenizer (e.g., a Silverson Model L4RT homogenizer (5/8" probe) set at 7000 RPM for about 12 seconds; a 60 second homogenization time would be too harsh at this homogenization speed) or a microfluidizer.

This process forms a second emulsion which is subsequently added to another solution containing an organic compound (e.g., PVA) with stirring (e.g., in a homogenizer or on a stir plate). This step causes the first organic solvent (e.g., dichloromethane) to be released and the microspheres to become hardened. Heat can alternatively be used to accelerate evaporation of the solvent. Slow release of the organic solvent (e.g., at room temperature) results in "spongy" particles having a highly porous structure throughout, while fast release (e.g., at elevated temperature) results in hollow-core microparticles. The latter solution can, for example, be 0.05% w/v PVA. If sugar or other compounds are added to the DNA, an equal concentration of the compound can be added to the third or fourth solution to equalize osmolarity, effectively decreasing the loss of nucleic acid from the microsphere during the hardening process. The resultant microparticles are washed several times with water to remove the organic compounds. Particles can be passed through sizing screens to selectively remove those larger than the desired size. If the size of the microparticles is not crucial, one can dispense with the sizing step. After washing, the particles can either be used immediately or be lyophilized for storage.

Larger particles, such as those used for implantation, can be obtained by using less vigorous emulsification conditions when making the first emulsion, as has already been described above at length. For example, larger particles could be obtained by altering the concentration of the polymer, altering the viscosity of the emulsion, altering the particle size of the first emulsion (e.g., larger particles can be made by decreasing the pressure used while creating the first emulsion in a microfluidizer), or homogenizing with, for example, the Silverson homogenizer set at 5000 RPM for about 12 seconds.

The recovered microparticles can be suspended in an excipient without negatively affecting the amount of supercoiled plasmid DNA within the microspheres. Excipients are often used in drug formulation, and here provide for efficient microsphere resuspension, act to prevent settling, and retain the microspheres in suspension. According to analysis by gel electrophoresis, excipients (including Tween 80, mannitol, sorbitol, and carboxymethylcellulose) have no effect on DNA stability or supercoiling.

After recovery of the microspheres or suspension of the microspheres in an excipient, the samples can be frozen and lyophilized for future use.

### Characterization of Microparticles

The size distribution of the microparticles prepared by the above method can be determined with a COULTER™ counter. This instrument provides a size distribution profile and statistical analysis of the particles. Alternatively, the average size of the particles can be determined by visualization under a microscope fitted with a sizing slide or eyepiece.

If desired, the nucleic acid can be extracted from the microparticles for analysis by the following procedure. Microparticles are dissolved in an organic solvent such as chloroform or methylene chloride in the presence of an aqueous solution. The polymer stays in the organic phase, while the DNA goes to the aqueous phase. The interface between the phases can be made more distinct by centrifugation. Isolation of the aqueous phase allows recovery of the nucleic acid. To test for degradation, the extracted nucleic acid can be analyzed by HPLC or gel electrophoresis.

To increase the recovery of nucleic acid, additional organic solvents, such as phenol and chloroform, can be added to the dissolved microparticles, prior to the addition of the aqueous solution. Following addition of the aqueous solution, the nucleic acid enters the aqueous phase, which can easily be partitioned from the organic phase after mixing. For a clean interface between the organic and aqueous phases, the samples should be centrifuged. The nucleic acid is retrieved from the aqueous phase by precipitation with salt and ethanol in accordance with standard methods.

### Intracellular Delivery of Microparticles

Microparticles containing DNA are resuspended in saline, buffered salt solution, tissue culture medium, or other physiologically acceptable carrier. For *in vitro*/*ex vivo* use, the suspension of microparticles can be added either to cultured adherent mammalian cells or to a cell suspension. Following a 1-24 hour period of incubation, those particles not taken up are removed by aspiration or centrifugation over fetal calf serum. The cells can be either analyzed immediately or recultured for future analysis.

Uptake of microparticles containing nucleic acid into the cells can be detected by PCR, or by assaying for expression of the nucleic acid. For example, one could measure transcription of the nucleic acid with a Northern blot, reverse transcriptase PCR, or RNA mapping. Protein expression can be measured with an appropriate antibody-based assay, or with a functional assay tailored to the function of the polypeptide encoded by the nucleic acid. For example, cells expressing a nucleic acid encoding luciferase can be assayed as follows: after lysis in the appropriate buffer (e.g., cell lysis culture reagent, Promega Corp, Madison WI), the lysate is added to a luciferin containing substrate (Promega Corp) and the light output is measured in a luminometer or scintillation counter. Light output is directly proportional to the expression of the luciferase gene.

If the nucleic acid encodes a peptide known to interact with a class I or class II MHC molecule, an antibody specific for that MHC molecule/peptide complex can be used to detect the complex on the cell surface of the cell, using a fluorescence activated cell sorter (FACS). Such antibodies can be made using standard techniques (Murphy et al. Nature, Vol. 338, 1989, pp. 765-767). Following incubation with microparticles containing a nucleic acid encoding the peptide, cells are incubated for 10-120 minutes with the specific antibody in tissue culture medium. Excess antibody is removed by washing the cells in the medium. A fluorescently tagged secondary antibody, which binds to the first antibody, is incubated with the cells. These secondary antibodies are often commercially available, or can be prepared using known methods. Excess secondary antibody must be washed off prior to FACS analysis.

One can also assay by looking at T or B effector cells. For example, T cell proliferation, cytotoxic activity, apoptosis, or cytokine secretion can be measured.

Alternatively, one can directly demonstrate intracellular delivery of the particles by using nucleic acids which are fluorescently labeled, and analyzing the cells by FACS. Internalization of the fluorescently labeled nucleic acid causes the cell to fluoresce above background levels. Because it is rapid and quantitative, FACS is especially useful for optimization of the conditions for *in vitro* or *in vivo* delivery of nucleic acids. Following such optimization, use of the fluorescent label is discontinued.

If the nucleic acid itself directly affects cellular function, e.g., if it is a ribozyme or an antisense molecule, or is transcribed into one, an appropriate functional assay can be utilized. For example, if the ribozyme or antisense nucleic acid is designed to decrease expression of a particular cellular protein, the expression of that protein can be monitored.

### In Vivo Delivery of Microparticles

Microparticles containing nucleic acid can be injected into mammals intramuscularly, intravenously, intraarterially, intradermally, intraperitoneally, intranasally, or subcutaneously, or they can be introduced into the gastrointestinal tract or the respiratory tract, e.g., by inhalation of a solution or powder containing the microparticles. Expression of the nucleic acid is monitored by an appropriate method. For example, expression of a nucleic acid encoding an immunogenic protein of interest is assayed by looking for an antibody or T cell response to the protein.

Antibody responses can be measured by testing serum in an ELISA assay. In this assay, the protein of interest is coated onto a 96 well plate and serial dilutions of serum from the test subject are pipetted into each well. A secondary, enzyme-linked antibody, such as anti-human, horseradish peroxidase-linked antibody, is then added to the wells. If antibodies to the protein of interest are present in the test subject's serum, they will bind to the protein fixed on the plate, and will in turn be bound by the secondary antibody. A substrate for the enzyme is added to the mixture and a colorimetric change is quantitated in an ELISA plate reader. A positive serum response indicates that the immunogenic protein encoded by the microparticle's DNA. was expressed in the test subject, and stimulated an antibody response. Alternatively, an ELISA spot assay can be employed.

T cell proliferation in response to a protein following intracellular delivery of microparticles containing nucleic acid encoding the protein is measured by assaying the T cells present in the spleen, lymph nodes, or peripheral blood lymphocytes of a test animal. The T cells obtained from such a source are incubated with syngeneic APCs in the presence of the protein or peptide of interest. Proliferation of T cells is monitored by uptake of ³H-thymidine, according to standard methods. The amount of radioactivity incorporated into the cells is directly related to the intensity of the proliferative response induced in the test subject by expression of the microparticle-delivered nucleic acid. A positive response indicates that the microparticle containing DNA encoding the protein or peptide was taken up and expressed by APCs *in vivo.*

The generation of cytotoxic T cells can be demonstrated in a standard ⁵¹Cr release assay. In these assays, spleen cells or peripheral blood lymphocytes obtained from the test subject are cultured in the presence of syngeneic APCs and either the protein of interest or an epitope derived from this protein. After a period of 4-6 days, the effector cytotoxic T cells are mixed with ⁵¹Cr-labeled target cells expressing an epitope derived from the protein of interest. If the test subject raised a cytotoxic T cell response to the protein or peptide encoded by the nucleic acid contained within the microparticle, the cytotoxic T cells will lyse the targets. Lysed targets will release the radioactive ⁵¹Cr into the medium. Aliquots of the medium are assayed for radioactivity in a scintillation counter. Assays, such as ELISA, can also be used to measure cytokine profiles.

The following are examples of the practice of the invention. They are not to be construed as limiting the scope of the invention in any way.

### EXAMPLE 1: Incorporation of DNA: Analysis of Particle Size and DNA Integrity

### Preparation of DNA for incorporation

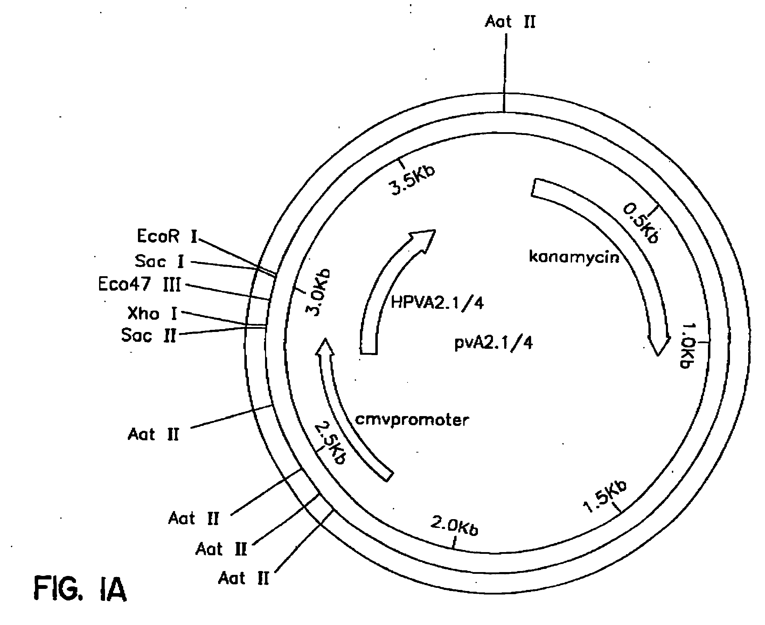

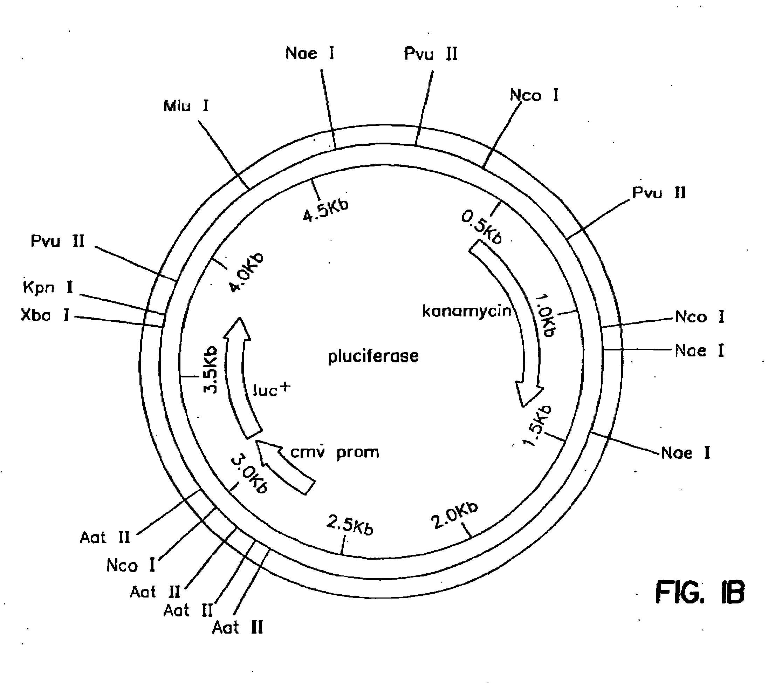

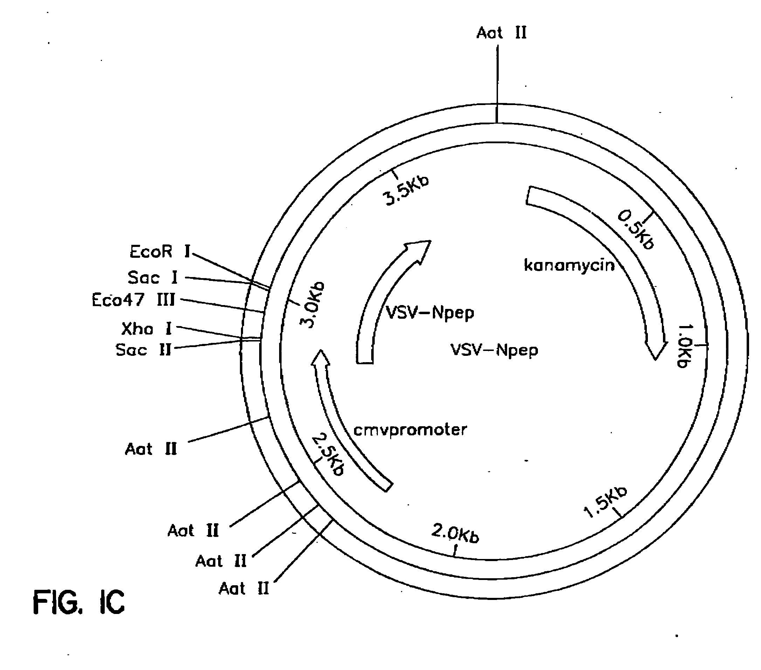

Plasmid DNA was prepared by standard methods using MEGA-PREP™ Kit (Qiagen) according to the manufacturer's instructions. An endotoxin-free buffer kit (Qiagen) was used for all DNA manipulations. The DNA was resuspended in distilled, deionized, sterile water to give a final concentration of 3 µg/µl. Fig. 1 shows plasmid maps of DNA expression vectors encoding a) luciferase, b) a vesicular stomatitis virus (VSV) peptide epitope termed VSV-Npep, and c) a human papilloma virus (HPV) peptide epitope termed A2.1/4.

### Association of DNA with PLGA

200 mg of poly-lactic-*co*-glycolic acid (PLGA) (Aldrich, 65:35 ratio of lactic acid to glycolic acid) was dissolved in 5-7 ml of methylene chloride. 300 µl of the DNA solution prepared above, containing 900 µg DNA, was added to the PLGA solution. The mixture was sonicated in a Model 550 SONIC DISMEMBRATOR™ (Fisher Scientific) on setting #3 for 5-60 seconds, and the resulting emulsion was analyzed. An emulsion verified to contain particles of desired size having DNA of satisfactory integrity (as determined below) was added to a beaker containing 50 ml aqueous 1% w/v polyvinyl alcohol (PVA) (mw range: 30-70 kdal). The mixture was homogenized in a POWERGEN™ homogenizer (Fisher scientific) set at 3000-9000 RPM for 5-60 seconds. Again, the DNA integrity was analyzed. In the cases where the DNA was found to be sufficiently intact, the resulting second emulsion was transferred into a second beaker containing 100 ml aqueous 0.05% PVA, with constant stirring. The stirring was continued for 2-3 hours.

The microparticle solution was poured into a 250 ml centrifuge tube and spun at 2000 rpm for 10 minutes. The contents of the tubes were decanted and the sedimented particles were resuspended in 100 ml deionized water. After repeating the centrifugation and decanting steps, the particles were frozen in liquid nitrogen and finally lyophilized until dry.

### Analysis of Microparticle size profile

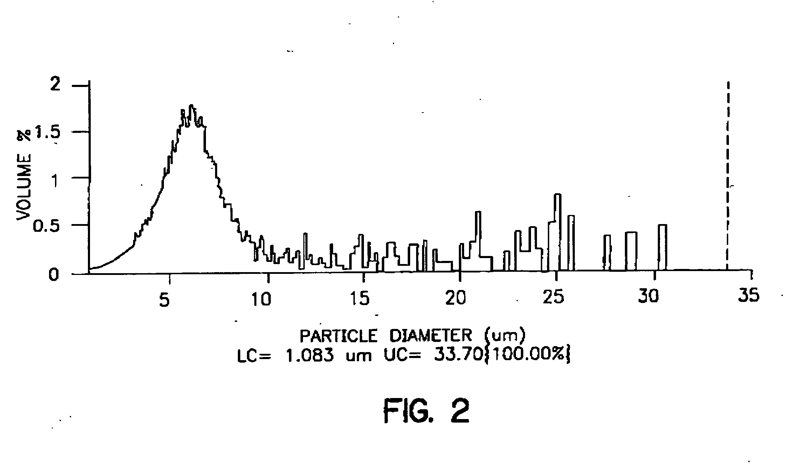

5 mg of the lyophilized microparticles were resuspended in 200 µl water. The resulting suspension was diluted to about 1:10,000 for analysis with a COULTER™ counter. Fig. 2 is a print-out from the COULTER™ counter which indicates that approximately 85% of the microparticles were between 1.1 and 10 µm in diameter.

### Determination of DNA Integrity

2-5 µg of the microparticles were wet with 10 µl water in an EPPENDORF™ tube. 500 µl chloroform was added with thorough mixing to dissolve the polymeric matrix. 500 µl water was added, again with mixing. The resulting emulsion was centrifuged at 14,000 rpm for 5 minutes. The aqueous layer was transferred to a clean EPPENDORF™ tube, along with 2 volume equivalents of ethanol and 0.1 volume equivalents of 3M aqueous sodium acetate. The mixture was centrifuged at 14,000 rpm for 10 minutes. After aspiration of the supernatant, the pelleted DNA was resuspended in 50 µl water. 5 µg DNA was electrophoresed on a 0.8% agarose gel next to a standard containing the input DNA. The DNA on the gel was visualized on a UV light box. Comparison with the standard gives an indication of the integrity of the microparticles' DNA. The microparticle formation procedure was deemed successful if the incorporated DNA retained a high percentage of supercoiled DNA relative to the input DNA.

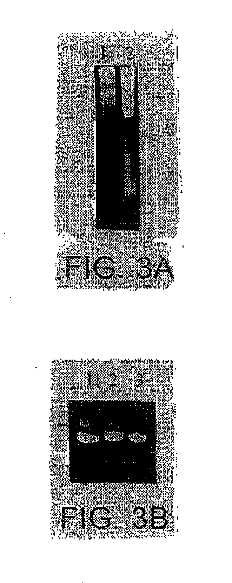

As indicated in Figs. 3A and 3B, homogenization speed and duration are inversely related to DNA integrity. Fig. 3A depicts the DNA isolated from microparticles prepared by homogenization at 7000 rpm for 1 minute (lane 1), and supercoiled input DNA (lane 2). Fig. 3B shows DNA isolated from microparticles prepared by homogenization at 7000 rpm for 5 seconds (lane 1), DNA isolated from microparticles prepared by homogenization at 5000 rpm for 1 minute (lane 2), and supercoiled input DNA (lane 3).

### EXAMPLE 2: Preparation of DNA and Microspheres DNA preparation

500 ml bacterial cultures were poured into one liter centrifuge bottles. The cultures were centrifuged at 4000 rpm at 20°C for 20 minutes. The media were poured off from the pelleted bacteria. The bacterial pellet was completely resuspended in 50 ml buffer p1 (50mM Tris-HCl, pH 8.0; 10mM EDTA; 100 µg/ml RNAse), leaving no clumps. 50 ml of buffer P2 (200 mM NaOH, 1% SDS) was added with gentle swirling, and the suspensions were incubated at room temperature for five minutes. 50 ml of buffer P3 (3.0 M potassium acetate, pH 5.5, chilled to 4°C) was added with immediate, gentle mixing. The suspensions were incubated on ice for 30 minutes, then centrifuged at 4000 rpm at 4°C for 30 minutes.

A folded, round filter was wetted with water. When the centrifugation was complete, the supernatant was immediately poured through the filter. The filtered supernatant was collected in a clean 250 mL centrifuge bottle.

15 mL of Qiagen ER buffer was added to the filtered lysate, mixing by inverting the bottle 10 times. The lysate was incubated on ice for 30 minutes.

A Qiagen-tip 2500 column was equilibrated by applying 35 mL QBT buffer (750 mM sodium chloride; 50 mM HOPS, pH 7.0; 15% isopropanol; and 0.15% triton X-100). The column was allowed to empty by gravity flow. The incubated lysate was applied to the column and allowed to enter by gravity flow. The column was washed with 4 x 50 ml Qiagen Endofree QC buffer (1.0 M NaCl; 50 mM MOPS, pH 7.0; 15% isopropanol). The DNA was eluted from the column with 35 ml of QN buffer (1.6 M NaCl,; 50 mM MOPS, pH 7.0; 15% isopropanol) into a 50 ml polypropylene screwcap centrifuge tube. The DNA suspension was split into two tubes by pouring approximately 17.5 ml of the suspension into a second 50 ml screwcap tube.

Using a sterile 10 ml pipet, 12.25 ml isopropanol was added to each tube. The tubes were closed tightly and thoroughly mixed. The contents of each tube were poured into 30 ml Corex (VWR) centrifuge tubes. Each Corex tube was covered with PARAFILM®. The tubes were centrifuged at 11,000 rpm at 4°C for 30 minutes.

The supernatant was aspirated from each tube and the pellet was washed with 2 ml 70% ethanol. The ethanol was aspirated off. The pellet was air dried for 10 minutes, then resuspended in 0.5-1.0 ml water, and transferred to a sterile 1.5 ml microfuge tube.

### Preparation of microspheres

200 mg PLGA was dissolved in 7 ml methylene chloride in a 14 ml culture tube. A Fisher Scientific PowerGen 700 homogenizer equipped with a 7 mm mixing head was set to setting 6 and the speed 4.5. A Fisher Scientific sonic Dismembrator 550 sonicator was set to setting 3.

1.2 mg of DNA in 300 µl H₂O was added to the PLGA solution and the resulting mixture was sonicated for 15 seconds. 50 ml of 1.0% PVA was poured into a 100 ml beaker and placed under the homogenizer. The homogenizer probe was immersed until it was about 4 mm from the bottom of the beaker and the homogenizer was supplied with power. The DNA/PLGA mixture was immediately poured into the beaker and the resultant emulsion was homogenized for 10 seconds. The homogenate was poured into the beaker containing 0.05% PVA.

The resulting emulsion was stirred for two hours, poured into a 250 ml conical centrifuge, and spun at 2000 rpm for 10 minutes. The pelleted microspheres were washed with 50 ml water, transferred to a 50 ml polypropylene centrifuge tube, and spun at 2000 rpm for 10 minutes. The pellet was washed with another 50 ml water and spun again at 2000 rpm for 10 minutes. The pellet was frozen in liquid nitrogen, then lyophilized overnight .

### Extraction of DNA from microspheres for gel analysis

1 ml of microspheres suspended in liquid were removed to a 1.5 ml microfuge tube and spun at 14,000 rpm for 5 minutes. Most of the supernatant was removed. 50 µl of TE buffer (10 mM Tris-BCl, pH 8.0; 1 mM EDTA) was added and the microspheres were resuspended by flicking the side of the tube.

To isolate freeze-dried or vacuum-dried microspheres, 2-4 mg microspheres were weighed out into a 1.5 ml microfuge tube. 70 µl TE buffer was added, and the microspheres were resuspended.

200 µl chloroform was added and the tubes were vigorously, but hot violently, shaken for two minutes to mix the aqueous and organic layers. The tubes were centrifuged at 14,000 rpm for 5 minutes. 30 µl of the aqueous phase was carefully removed to a new tube.

### Pico Green and Gel Analysis of Microspheres

3.5-4.5 mg microspheres were weighed out into a 1.5 ml microfuge tube. 100 µl DMSO was added to each tube, and the tubes were rotated at room temperature for 10 min. The samples were removed from the rotator and visually inspected to verify that the samples were completely dissolved. Where necessary, a pipet tip was used to break up any remaining clumps. None of the samples were allowed to remain in DMSO for more than 30 minutes.

For each sample to be tested, 990 µl TE was pipetted into three separate microfuge tubes. 10 µl of the DMSO/microsphere solution was pipetted into each 990 µl TE with mixing. The mixtures were centrifuged at 14,000 rpm for 5 minutes.

For each sample, 1.2 ml TE was aliquoted into a 5 ml round bottom snap cap centrifuge tube. 50 µl of the 1 ml TE/DMSO/microsphere mixture to the 1.2 ml TE. 1.25 ml of pico green reagent was added to each tube, and the fluorescence was measured in a fluorimeter.

### EXAMPLE 3: Alcohol Precipitation

### Ethanol precipitation

DNA was prepared as in Example 2. Three samples, each containing 1.2 mg DNA, were precipitated by the addition of 0.1 vol 3 M sodium acetate and 2 volumes of ethanol. The DNA was resuspended in water to a final concentration of 4 mg/ml. DNA in two of the samples was resuspended immediately before use, and DNA in the third sample was resuspended and then rotated for 4 hours at ambient temperature. Control DNA at 4mg/ml was not ethanol precipitated.

Each of the four samples was encapsulated into microspheres by the procedure described in Example 2. The amount of DNA per mg of microspheres was determined by pico green analysis, as described in Example 2. The following results were obtained:

| Sample | mg of MS | µg DNA/mg MS | % incorp. | % incr. |
|---|---|---|---|---|
| Ethanol, 0 hr #1 | 4.66 | 3.37 | 56 | 44 |
| Ethanol, 0 hr #2 | 4.45 | 4.91 | 82 | 62 |
| Ethanol, 4 hr | 3.96 | 4.30 | 72 | 57 |
| Unprecip. | 3.97 | 1.85 | 31 | - |

The results indicate that ethanol precipitation of DNA prior to encapsulation in microspheres resulted in increased incorporation ranging from 31% to greater than 56%, representing a 44-62% increase in the amount of encapsulated DNA.

The following experiments verify that the ethanol-precipitation effects observed above are independent of DNA preparation procedures.

DNA was prepared at three different facilities. Sample #1 was prepared as in Example 2. Sample #2 was prepared as in Example 2, but without the addition of ER-removal buffer. Sample #3 was prepared in a scaled-up fermentation manufacturing run. The three DNA samples were representative of two different plasmids (DNA-1 and DNA-3 were identical) of sizes 4.5 kb and 10 kb. The three DNA samples were tested for the enhancement of encapsulation efficiency by ethanol precipitation. Three samples of DNA, each containing 1.2 mg, were precipitated by the addition of 0.1 vol 3 M sodium acetate and 2 volumes ethanol. The DNA was resuspended in water at a concentration of 4 mg/ml. Three control DNA samples, at 4mg/ml, were not ethanol precipitated.

Each of the samples was encapsulated by the procedure described in Example 2.

The amount of DNA per mg of microspheres was determined by pico green analysis as described in Example 2. The following results were obtained:

| Sample | mg of MS | µg DNA/mg MS | % incorp. | % incr. |
|---|---|---|---|---|
| #1 eth. ppt. | 3.35 | 3.10 | 67 | 59 |
| #2 eth. ppt. | 4.45 | 4.91 | 66 | 47 |
| #3 eth. ppt. | 3.34 | 2.65 | 48 | 29 |
| #1 unppt. | 3.38 | 1.95 | 42 | - |
| #2 unppt. | 3.35 | 1.80 | 45 | - |
| #3 unppt. | 3.33 | 1.81 | 37 | - |

The data show that ethanol precipitation increased the amount of DNA encapsulated in microspheres by 29-59%. The effect was demonstrated to hold regardless of size and preparation technique.

### Isopropanol vs. ethanol precipitation

Plasmid DNA was precipitated with ethanol or isopropanol, then resuspended in water for 4 hours or 16 hours. Control DNA was not precipitated. Microspheres were made according to the protocol in Example 2. The following results were obtained:

| Sample | mg of MS | µg DNA/mg MS | % incorp. | % incr. |
|---|---|---|---|---|
| unppt. #1 | 4.43 | 0.99 | 17 | - |
| unppt. #2 | 4.30 | 0.99 | 17 | - |
| eth. ppt. #1 16 hr | 4.26 | 2.12 | 37 | 118 |
| eth. ppt. #2 16 hr | 4.34 | 1.66 | 31 | 82 |
| isopro. ppt. #1 16 hr | 4.60 | 1.71 | 31 | 82 |
| isopro. ppt. #2 16 hr | 4.90 | 1.72 | 32 | 88 |
| eth. ppt. #1 4 hr | 4.65 | 2.22 | 42 | 147 |
| eth. ppt. #2 4 hr | 4.27 | 1.69 | 30 | 76 |
| isopro. ppt. #1 4 hr | 4.55 | 1.41 | 25 | 47 |
| isopro. ppt. #2 4 hr | 4.30 | 2.78 | 46 | 170 |

These data demonstrate that alcohol precipitation increased the encapsulation efficiency of DNA, independent of the type of alcohol used to precipitate DNA and independent of the time following DNA precipitation. Conductivity

The conductivities of the ethanol-precipitated and non-precipitated DNA samples were determined using a conductivity meter. It was found that precipitation of the DNA led to a decrease in the amount of salt present. The conductivity without ethanol precipitation was 384 µΩ, while the conductivity after ethanol precipitation was 182 µΩ. Thus, alcohol precipitation, or any other means of salt/contaminant removal is likely to increase encapsulation efficiency. It therefore appears that treatments that render DNA free from contaminants are likely to increase the efficiency of DNA encapsulation.

DNA was then ethanol precipitated or precipitated in the presence of 0.4M NaCl and 5% hexadecyltrimethylammonium bromide (CTAB). The DNA was then encapsulated as described above. The DNA was extracted and analyzed by agarose gel electrophoresis. The results indicated that precipitation of the DNA with CTAB led to a marked increase in the amount of supercoiled DNA within the microspheres.

### EXAMPLE 4: Addition of Stabilizer Compounds

### TE buffer

Plasmid DNA was resuspended in TE buffer following ethanol-precipitation, in an attempt to increase DNA stability. The microspheres were then prepared as described in Example 2. DNA was extracted from the microspheres and analyzed by agarose gel electrophoresis. One lane was loaded with the input plasmid (pIiPLPLR); another lane with the plasmid DNA following ethanol precipitation, resuspension in water, and encapsulation in microspheres; and still another, lane with the plasmid DNA following ethanol precipitation, resuspension in TE buffer, and encapsulation in microspheres. The results indicated that the amount of supercoiled DNA within microspheres was increased by resuspension in TE buffer.

Two other plasmids, designated pbkcmv-n-p and E3PLPLR, were subjected to the conditions described above. This experiment confirmed that the two other plasmids were also stabilized by the TE buffer.

The following experiment was conducted to determine the timing of the TE effect. 2 g PLGA was dissolved in 18 ml methylene chloride. 500 µg DNA was ethanol-precipitated and dissolved in 3.6 ml TE or water. The two solutions were mixed by inverting several times and then sonicated in the Fisher apparatus (see Example 2) on setting 3 for 10 seconds with a 1/8" microtip. At various times after sonication (i.e., 5, 15, 30, 45, and 60 minutes), a 1 ml sample was removed from each tube, 100 µl water was added, the sample was centrifuged in an Eppendorf centrifuge, and the top layer of the centrifugated sample removed to a separate tube. The samples were then analyzed by gel electrophoresis.

The results indicated that TE buffer acted to stabilize the DNA early in the encapsulation process, during formation of the oil in water emulsion.

To determine the effect of Tris and/or EDTA in the TE buffer, DNA was resuspended in water, TE buffer, 10 mM TRIS, or 1 mM EDTA prior to encapsulation in microspheres by the method of Example 2. The DNA was extracted from the microspheres and analyzed on an agarose gel. Tris and EDTA were each found to be similar to the complete TE buffer in their ability to protect DNA during the encapsulation process and during lyophilization.

An experiment was carried out to determine the effect of pH on encapsulation (the pH of the EDTA, Tris, and TE solutions in the previous experiment were all similar). Microspheres were made by encapsulating DNA that had been ethanol precipitated and resuspended in Tris of different pH, or in phosphate buffered saline (PBS). The DNA was extracted after lyophilization of the particles, and analyzed on agarose gel. The results indicated that there was a significant pH effect on the stability of encapsulated DNA. Resuspension of the DNA in water (pH 6.5), PBS (pH 7.3), and Tris (pH 6.8) all led to a decrease in the ratio of supercoiled DNA relative to total DNA within the microspheres. Increasing the pH to 7.5 or higher had a positive effect on the amount of supercoiling, suggesting that basic pH levels are important for maintaining DNA stability. Increased pH also had an effect on encapsulation efficiency:

| SAMPLE | mg of MS | µg DNA/mg MS | % incorp. |
|---|---|---|---|
| Tris pH 6.8 | 2.42 | 2.77 | 55.5 |
| Tris pH 7.5 | 2.52 | 2.73 | 54.6 |
| Tris pH 8.0 | 2.49 | 3.29 | 65.9 |
| Tris pH 9.9 | 2.46 | 3.81 | 76.3 |
| water | 2.46 | 2.48 | 49.7 |
| PBS pH 7.3 | 2.49 | 0.55 | 11 |
| TE pH 8.0 | 2.52 | 2.22 | 44.3 |

### Other buffer compounds

Borate and phosphate buffers were also tested for their effect on the quality of encapsulated DNA. DNA was ethanol precipitated, resuspended in various buffer solutions, and encapsulated according to the procedure of Example 2. The DNA was extracted from the microspheres and analyzed by agarose gel electrophoresis. TE, BE, and PE all afforded greater than 50% supercoiling in the encapsulated DNA. An added benefit to DNA was also discovered, resulting from EDTA in the presence of Tris, borate, or phosphate.

### Other stabilizer compounds

In addition to buffers, other compounds were tested for their ability to protect the DNA during the encapsulation procedure. Plasmid DNA was ethanol-precipitated and resuspended in water or a solution of dextran sulfate. Microspheres were then prepared according to the method of Example 2. DNA was extracted from the microspheres before and after lyophilization and analyzed by agarose gel electrophoresis.

The results suggested that the addition of a stabilizer led to encapsulation of more supercoiled DNA than did resuspension of DNA in water alone. The greatest improvement in DNA structure was observed with a 10% dextran sulfate solution. Protection apparently occurred at two levels. An effect of dextran sulfate was seen on DNA pre-lyophilization, as, following encapsulation, a greater proportion of DNA remained in the supercoiled state with increasing amounts of dextran sulfate. The protection rendered by the stabilizer also occurred during the lyophilization procedure, since the presence of the stabilizer during this process increased the percentage of DNA remaining in the supercoiled state.

To determine whether or not the effects of TE and other stabilizers were additive, ethanol-precipitated DNA was resuspended in TE or water, with or without a solution of another stabilizer (e.g., sucrose, dextrose, CTAB, cyclodextrin, or dextran). Microspheres were prepared according to the method of Example 2. DNA was extracted from the microspheres and analyzed by agarose gel electrophoresis.

The results demonstrated that resuspending DNA in a stabilizer/TE solution is better than or equivalent to the use of TE alone, insofar as a greater percentage of DNA remains in the supercoiled state after encapsulation under these conditions.

Stabilizers were also added in combination, to determine whether or not the stabilizer effects are additive. DNA was ethanol-precipitated and resuspended in various stabilizer solutions. The DNA was encapsulated as described in Example 2, extracted, and analyzed by agarose gel electrophoresis. The results indicated that combinations of stabilizers can be used to increase the amount of encapsulated, supercoiled DNA.

### EXAMPLE 5: Addition of Excipients

To determine whether or not excipient compounds have an adverse effect on encapsulated plasmid DNA, microspheres were prepared from ethanol-precipitated DNA following the protocol in Example 2, with the exception that prior to lyophilization, the microspheres were resuspended in solutions containing excipients. Each sample was then frozen and lyophilized as in Example 2. The final concentration of the excipients in the microspheres upon resuspension at 50 mg/ml was 0.1% Tween 80, 5% D-sorbitol, 5% D-mannitol, or 0.5% carboxymethylcellulose (CMC). DNA was extracted from the microspheres and analyzed on an agarose gel.

The results illustrated that addition of excipients prior to lyophilization did not significantly affect DNA stability or the degree of supercoiling.

### EXAMPLE 6: In Vitro Cell Studies

### In Vitro Phagocytosis of DNA-containing Microparticles

Into each of two wells of a six-well tissue culture dish, about 10⁶ macrophages were plated in 3 ml RPMI medium containing 10% fetal calf serum. 5 mg of the microparticles containing DNA encoding luciferase were resuspended in 200 µl saline solution, and 50 µl of the resulting suspension was added to one of the wells containing macrophages. The plate was incubated at 37°C for 1-6 hours. Side vs. forward scatter (i.e., intracellular complexity vs. size) of the cells was analyzed by FACS using a Becton Dickinson FACS instrument.

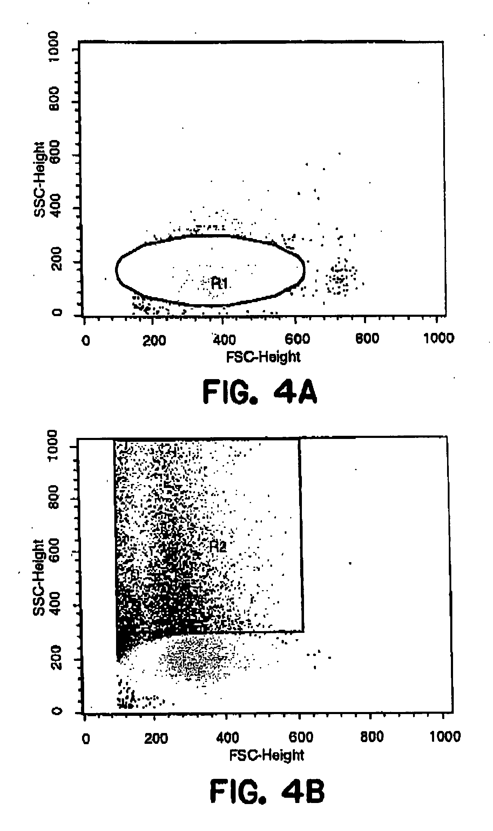

Fig. 4 shows the results. Cell populations that have not phagocytosed are found in region R1. Phagocytosing cells remain the same size (FSC profile), but demonstrate an increased side scatter profile. These cells are found in region R2.

### Measurement of DNA Expression Following Phagocytosis

Into two wells of a 24-well tissue culture dish, about 2.5×10⁵ macrophages were plated in 1 ml RPMI medium containing 10% fetal calf serum. The plate was incubated at 37°C for 6 hours. 1 mg of the lyophilized microparticles containing DNA encoding luciferase was resuspended in 400 µl saline solution. 6 µl of the resulting suspension was added to one of the wells containing macrophages, and 25 µl of suspension was added to the other. The plate was incubated at 37°C for 4 hours. The medium, including the microparticles, was removed and fresh medium added to the cells. The plate was again incubated at 37°C for 1-5 days. The cells were harvested into a tube and spun at 1,500 RPM for 5 minutes. The pelleted cells were resuspended in 100 µl of 1X Cell Lysis Buffer (Promega) in an EPPENDORF™ tube. The mixture was centrifuged at 14,000 RPM for 5 minutes in order to precipitate out any cell debris. The cell lysate was assayed by adding 5 µl of the supernatant to 100 µl of luciferase substrate (Promega) and measuring the light output on a TOPCOUNT™ combination luminometer/scintillation counter (Packard Instruments).

The data for this experiment are provided in Table 5. They indicate that cells phagocytosing microparticles that contain, for example, luciferase DNA, do in fact express the DNA. Thus, DNA integrity and functionality are confirmed. The data also indicate that the uptake of the microparticles by phagocytosis does not prevent the DNA from reaching the nucleus.

**TABLE 5: Phagocytosis of encapsulated DNA leads to expression of a luciferase reporter gene construct.**

| **MICROPARTICLES CONTAINING:** | | | | |
|---|---|---|---|---|
| | **Luciferase DNA** | | **Control DNA** | |
| | **25 µl** | **6 µl** | **25 µl** | **6 µl** |
| **Day 1** | 1257 | 168 | 103 | 245 |
| **Day 2** | 2632 | 492 | 107 | 133 |
| **Day 3** | 3400 | 507 | 80 | 93 |
| **Day 5** | 763 | 310 | 90 | 90 |

| | | | | |
|---|---|---|---|---|
| Data given in counts per 0.01 minute | | | | |

### EXAMPLE 7: In Vivo Cell Studies

### In Vivo Expression of Incorporated DNA

45 mg of luciferase cDNA in microparticles was resuspended in 250 µl saline solution. 40 µl of the resulting suspension was injected into each tibialis anterior muscle of a mouse. Seven days later, each tibialis anterior was dissected and placed in an EPPENDORF™ tube on dry ice. Using a mortar and pestle cooled with dry ice, each tibialis anterior muscle was ground into a powder, then return to the EPPENDORF™ tube. 500 µl 1X cell lysis buffer (Promega) was added. The tube was shaken upside-down on a vortex mixer at 4°C for 15 minutes. The tube and its contents were frozen in liquid nitrogen, then thawed to 37°C. The freeze/thaw cycle was repeated two more times. The tube was centrifuged 14,000 RPM for 10 minutes. The supernatant was transferred to a new tube and centrifuged again for 5 minutes. To assay for expression, 20 µl of the supernatant was added to 100 µl of luciferase substrate (Promega) and the light output was measured on a TOPCOUNT™ combination luminometer/scintillation counter (Packard Instruments).

The data for this experiment are provided in Table 6. They indicate that muscle cells can express DNA released from microparticles. Since these cells are not known to phagocytose, this is an example of depot effect.

**TABLE 6: Expression of encapsulated luciferase DNA in murine muscles**

| | |
|---|---|
| **Muscle 1** | 2 x 10⁵ |
| **Muscle 2** | 8 x 10⁴ |
| **Muscle 3** | 1 x 10⁶ |
| **Muscle 4** | 6 x 10⁵ |
| **Control** | 2 x 10² |

| | |
|---|---|
| Data given in counts per 0.01 minute | |

### Generation of Cytotoxic T cells Following Injection of Microparticles containing DNA

90 mg of microparticles containing DNA encoding VSV-Npep was resuspended in 900 µl of saline solution. 60 mg of microparticles containing control vector DNA was resuspended in 600 µl of saline solution. 300 µg VSV-Npep plasmid DNA was resuspended in 300 µl of saline solution. 300 µg control vector DNA was resuspended in 300 µl of saline solution. 150 µg of the VSV-N peptide was resuspended in incomplete Freund's adjuvant (IFA).

The five suspensions were injected intraperitoneally, intramuscularly, or subcutaneously, according to the following regimen:
1. Intraperitoneal: A first group of 3 mice was injected intraperitoneally with 100 µl of microparticles containing VSV-Npep DNA (Group 1). A second group of 3 mice was injected with 100 µl of microparticles containing control vector DNA (Group 2).
2. Intramuscular: (into each tibialis anterior muscle): A third group of 3 mice was injected intramuscularly with 100 µl of microparticles containing, VSV-Npep DNA (Group 3). A fourth group of 3 mice was injected with 100 µl microparticles containing control vector DNA (Group 4). A fifth group of 3 mice was injected with 50 µg/leg VSV-Npep plasmid DNA (i.e., in the absence of microparticles) (Group 5). A sixth group of 3 mice was injected with 50 µg/leg control vector plasmid DNA (Group 6).
3. Subcutaneous: A seventh group of 3 mice was injected subcutaneously with loo µl of microparticles containing VSV-Npep DNA (Group 7). An eighth group of 3 mice was injected with 50 µg VSV-N peptide/IFA (Group 8).

After two weeks, groups 5, 6, and 8, which received either synthetic peptide or DNA without microparticles, were injected again. Groups 1-4 and 7, which initially received microparticles, were not reinjected.

Seven days after the last set of injections, the murine spleens were harvested. Single cell suspensions were generated by standard methods, the red blood cells were lysed, and the remaining cells were resuspended in RPMI with 10% fetal calf serum to give a final concentration of 4x10⁶ effector cells/ml. Half of the cells from each group were then incubated at 37°C for 6 days with an equal number of peptide-pulsed syngeneic stimulator cells which had been previously treated with mitomycin C. The remaining cells were incubated with 50 µM peptide alone.

After the second day of incubation, 0.1 volume equivalents of IL-2-containing supernatant, derived from cells incubated in ConA, was added. After the sixth day of incubation, the effector cells were harvested and incubated in 96-well round-bottom plates containing ⁵¹Cr-labeled, peptide-pulsed target cells at 37°C for 5 hours. The effector-to-target ratios for the wells ranged from 200:1 down to 1:1.

To determine the level of maximal lysis, 20 µl of aqueous 10% sodium dodecyl sulfate (SDS) was added to certain wells containing only target cells. To determine the level of spontaneous lysis, certain wells were incubated with media alone (i.e., target cells but no effector cells). Specific lysis is calculated as follows: [(experimental lysis)-(spontaneous lysis)/(maximal lysis)-(spontaneous lysis)] × 100 = specific lysis.

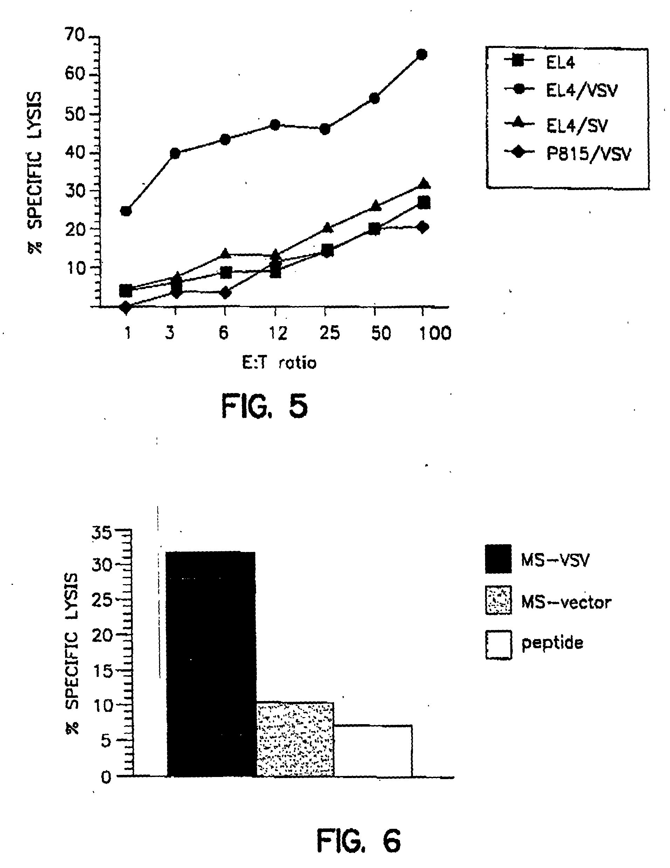

The results are shown in Figs. 5-9.

In the experiment associated with Fig. 5, effector cells from mice (Group 1) immunized intraperitoneally with microparticles containing DNA that encodes a peptide from the VSV-N protein were tested for cytolytic activity against various target cells. The VSV peptide binds to the mouse H-2K^{b} class I receptor. Syngeneic targets express the H-2K^{b} receptor while the allogeneic targets used in this experiment express the H-2K^{d} receptor.

CTL activity was tested on syngeneic targets (EL4) without peptide, syngeneic targets (EL4/VSV) labeled with the VSV peptide, syngeneic targets (EL4/SV) labeled with SV peptide (i.e., a non-specific peptide), and allogenic targets (P815/VSV) labeled with VSV peptide.

Because the allogeneic targets (PS15/VSV) do not express the H-2K^{b} receptor, they should not be recognized and lysed by the effector cells. Thus, P815 targets mixed with the VSV peptide are not lysed. Syngeneic targets (EL4) that do not have the VSV peptide are also not lysed. Syngeneic targets (EL4/SV) that express a peptide different from VSV are also not lysed. Only those targets (ETA/VSV) that have both the right MHC receptor and the right peptide are lysed.

Together, the data demonstrate that CTL activity can be elicited by immunization with microparticles containing DNA that encodes a VSV peptide, and the lysis is MHC restricted and peptide specific. In other words, only the right peptide with the right MHC receptor is recognized by the T cell receptor of the CTL generated by immunization in accordance with the invention. This demonstrated that the microparticles serve the desired function.

Next, the CTL response generated by immunizing mice subcutaneously with synthetic peptide (Group 8) was compared with the CTL response generated by immunizing mice intraperitoneally with microparticles containing DNA that encodes the VSV peptide (Groups 1 and 2). In Fig. 6 is shown the lysis obtained at a E:T ratio of 100:1 for CTL generated by immunizing the mice with either microparticles including DNA that encodes the VSV-N peptide (MS-VSV; Group 1), microparticles including control vector DNA that does not encode a VSV peptide (MS-vector; Group 2), or synthetic VSV-N peptide (peptide; Group 8). The targets were syngeneic (EL4) cells labelled with VSV peptide.

Mice immunized with the VSV-Npep DNA in microparticles (MS-VSV) generated a stronger CTL response (33% specific lysis) than mice immunized with control microparticles containing empty vector DNA (MS-vector) (10% specific lysis). Mice immunized with VSV-N peptide (peptide) generate a weaker CTL response than those immunized with microparticles containing VSV-Npep DNA (HS-VSV). Therefore, the microparticles served the desired function.

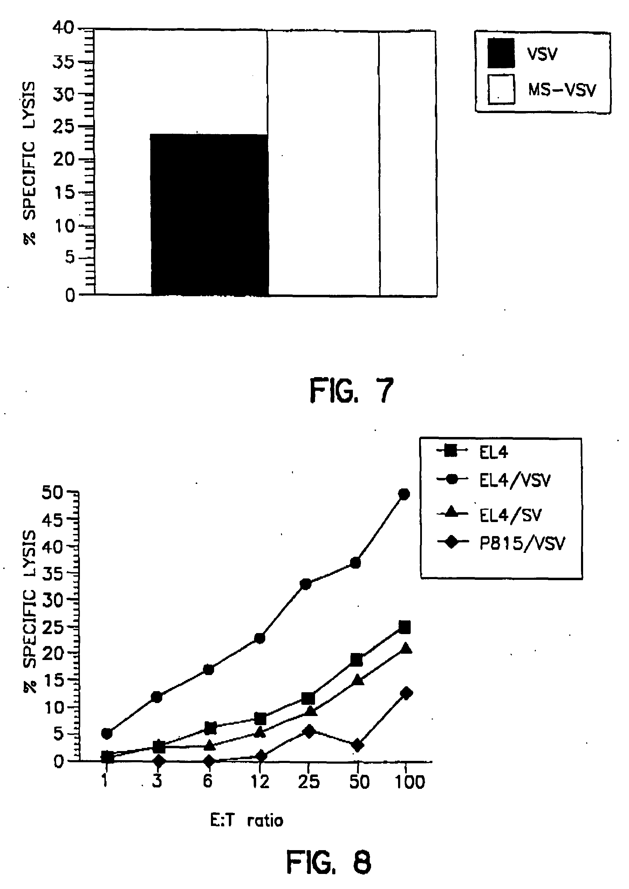

CTL responses in mice immunized intraperitoneally with VSV-Npep DNA contained in microparticles (MS-VSV) were compared with the CTL responses of mice immunized intramuscularly with "naked" VSV DNA (VSV). CTL responses in mice immunized with the microparticles containing DNA (MS-VSV; Group 1) were stronger than those in mice immunized with naked DNA (VSV; Group 5) at an E:T ratio of 3:1 (Fig. 7). The targets were syngeneic (EL4) calls labelled with VSV peptide. The mice which received naked DNA were immunized twice, while the mice immunized with microparticles were only given one treatment. The data in Fig. 7 therefore show that one injection of DNA in microparticles was more effective than two injections of a greater amount of naked DNA.

Fig. 8 shows the results of an experiment equivalent to that related in Fig. 5, with the exception that the injections were subcutaneous (Group 8 mice) instead of intraperitoneal. This experiment demonstrated that subcutaneous injections of microparticles containing VSV-Npep DNA are also effective for producing CTL responses.

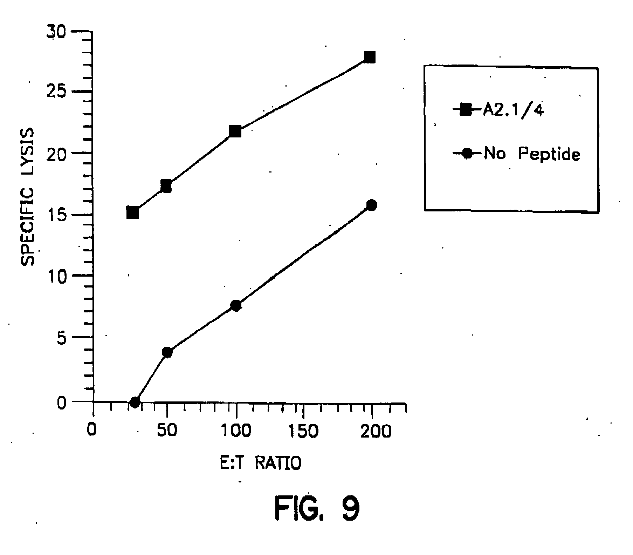

The experiment illustrated in Fig. 9 is also similar to that of Fig. 5, except that DNA encoding a different peptide was used in order to demonstrate that the results obtained were not unique to VSV-Npep DNA. HLA-A2 transgenic mice were immunized with microparticles containing DNA that encodes a peptide from human papillomavirus (HPV) E6 peptide. The HPV E6 peptide termed A2.1/4 binds to the human MHC receptor HLA-A2. The experiment assessed the ability of CTL effectors to lyse syngeneic targets (i.e., targets having the correct HLA receptor) that were either labeled with the correct HPV peptide (A2.1/4) or else unlabeled (no peptide). The E:T ratios are listed along the X-axis.

### EXAMPLE 8: Treatment with Microparticles Containing DNA

According to the procedure of example 1, microparticles are prepared containing DNA encoding a peptide having an amino acid sequence about 50% identical to PLP residues 170-191 (SEQ ID NO: 2). A multiple sclerosis patient whose T cells secrete excess T_{H}1 cytokines (i.e., IL-2 and γ-IFN) in response to autoantigens is injected intravenously with 100 µl to 10 ml of the microparticles. Expression of the PLP-like peptide by APCs results in the switching of the cytokine profile of the T cells, such that they instead produce T_{H}2 cytokines (i.e., IL-4 and IL-10) in response to autoantigens.

### EXAMPLE 9: Tolerizing with Microparticles Containing DNA

According to the procedure of example 1, microparticles are prepared containing DNA encoding a peptide having an amino acid sequence corresponding to MBP residues 33-52 (SEQ ID NO: 34). A mammal is injected subcutaneously with 1-500 µl of the microparticles. Expression of the MBP peptide by APCs results in the tolerization of T cells that recognize the autoantigen.

### EXAMPLE 10 : Implantation of Microparticles

A DNA molecule, including an expression control sequence operatively, linked to a sequence encoding both a trafficking sequence and a peptide essentially identical to myelin basic protein (MBP) residues 80-102 (SEQ ID NO: 1), is associated with a polymer to form microparticles, according to the procedure of example i. Particles smaller than 100 µm are removed. The polymeric constituent of the microparticle is poly-lactic-co-glycolic acid, where the ratio of lactic acid to glycolic acid is 65:35 by weight. The resulting microparticles are surgically implanted subcutaneously in a patient.

### EXAMPLE 11: Preparation of Microparticles containing Both DNA and Protein

Plasmid DNA is prepared by standard methods using MEGA-PREP™ Kit (Qiagen) according to the manufacturer's instructions. An endotoxin-free buffer kit (Qiagen) is used for all DNA manipulations. The DNA is resuspended in distilled, deionized, sterile water to give a final concentration of 3 µg/µl. Additionally, 0-40 mg of purified protein is added to about 1 ml of the DNA solution. A mass of gelatin, equal to about 20% of the mass of protein, is added.

Up to 400 mg of PLGA (i.e., at least ten times the mass of protein) is dissolved in about 7 ml methylene chloride. The DNA/protein solution is poured into the PLGA solution and homogenized or sonicated to form a first emulsion. The first emulsion is poured into about 50-100 ml of an aqueous solution of surfactant (e.g., 0.05% to 2% PVA by weight). The mixture is homogenized at about 3000-8000 RPM to form a second emulsion. The microparticles are then isolated according to the procedure of example 1.

### EXAMPLE 12: Treatment with Microparticles Containing Both DNA and Protein

Microparticles including both an antigenic protein having the conformational determinants necessary for induction of B cell response against hepatitis B virus (HBV) and DNA encoding the CTL epitope for HBV, are prepared according to the procedure of example 10. A patient infected or at risk of infection with HBV is immunized with the microparticles.

Slow release of the protein from non-phagocytosed microparticles leads to B cell recognition of the conformational determinants and subsequent secretion of antibody. Slow release of the DNA or phagocytosis of other microparticles causes APCs (1) to express the DNA of interest, thereby generating a T cell response; and (2) to digest the protein released from the microparticles, thereby generating peptides which are subsequently presented by class I or II molecules. Presentation by class I molecules promotes CTL response; presentation by class II molecules promotes both antibody and T cell responses, since T_{H} cells activated by the class II/peptide complexes secrete non-specific cytokines.

The results are elimination of HBV from the patient and continued prevention of production of virus within the patient's cells.

### EXAMPLE 13: Phagocytosis of Microspheres Containing Plasmid DNA by Murine Dendritic Cells

Microspheres were prepared by the procedure of Example 2, except that a fluorescent oligonucleotide was added during the encapsulation procedure. Splenic dendritic cells were isolated from mice and incubated with nothing, with fluorescent beads, or with the prepared microspheres. FACS analysis of the cells indicated that the fluorescent beads and the prepared microspheres were both phagocytosed. Moreover, the prepared microspheres did not fluoresce unless they had been ingested by the dendritic cells, suggesting that following phagocytosis, the microspheres became hydrated and degraded, allowing release the encapsulated DNA into the cell cytoplasm.

### Other Embodiments

It is to be understood that while the invention has been described in conjunction with the detailed description thereof, that the foregoing description is intended to illustrate and not limit the scope of the appended claims. Other aspects, advantages, and modifications are within the scope of the following claims.

## Claims

1. A preparation of microparticles, each of which comprises a polymeric matrix and nucleic acid, the polymeric matrix consisting essentially of one or more synthetic polymers having a solubility in water of less than about 1 mg/l, wherein
at least 90% of the microparticles have a diameter less than about 100 microns, and
the nucleic acid is an expression vector selected from the group consisting of RNA molecules, at least 50% of which are closed circles; and circular plasmid DNA molecules, at least 50% of which are supercoiled.

2. The preparation of claim 1, additionally comprising a stabilizer compound.

3. The preparation of claim 1,' wherein at least 90% of the microparticles have a diameter less than about 20 microns.

4. The preparation of claim 1, wherein at least 50% of the nucleic acid, by weight, consists of supercoiled plasmid DNA molecules.

5. A microparticle less than about 20 microns in diameter, comprising:
a polymeric matrix consisting essentially of one or more synthetic polymers having a solubility in water of less than about 1 mg/l; and
nucleic acid molecules, at least 50% of which are supercoiled DNA.

6. The microparticle of claim 5, wherein the polymeric matrix is biodegradable.

7. The microparticle of claim 5, wherein the polymeric matrix consists essentially of one synthetic, biodegradable copolymer.

8. The microparticle of claim 7, wherein the copolymer is poly-lactic-co-glycolic acid (PLGA).

9. The microparticle of claim 8, wherein the ratio of lactic acid to glycolic acid in the copolymer is within the range of about 1:2 to about 4:1 by weight.

10. The microparticle of claim 5, wherein the microparticle has a diameter of less than about 11 microns.

11. The microparticle of claim 5, wherein the microparticle is suspended in an aqueous solution.

12. The microparticle of claim 5 wherein the microparticle is in the form of a dry solid.

13. The microparticle of claim 5, wherein the nucleic acid molecule comprises an expression control sequence operatively linked to a coding sequence.

14. A microparticle less than about 20 microns in diameter, comprising:
a polymeric matrix; and
a nucleic acid molecule comprising an expression control sequence operatively linked to a coding sequence, wherein the coding sequence encodes an expression product selected from the group consisting of:
(1) a polypeptide at least 7 amino acids in length, having a sequence essentially identical to the sequence of
(a) a fragment of a naturally-occurring mammalian protein or
(b) a fragment of a naturally-occurring protein from an infectious agent which infects a mammal;
(2) a peptide having a length and sequence which permit it to bind to an MHC class I or II molecule; and
(3) said polypeptide or said peptide linked to a trafficking sequence.

15. The microparticle of claim 14, wherein the expression product comprises a peptide having a length and sequence which permit it to bind an MHC class I molecule.

16. The microparticle of claim 14, wherein the expression product comprises a peptide having a length and sequence which permit it to bind an MHC class II molecule.

17. The microparticle of claim 14, wherein the expression product (1) has an amino acid sequence that differs by no more than 25% from the sequence of a naturally occurring peptide recognized by a T cell; (2) is recognized by the T cell; and (3) alters the cytokine profile of the T cell.

18. The microparticle of claim 16, wherein the expression product comprises an amino acid sequence at least 50% identical to the sequence of a fragment of a protein selected from the group consisting of myelin basic protein (MBP), proteolipid protein (PLP), invariant chain, GAD65, islet cell antigen, desmoglein, α-crystallin, and β-crystallin, wherein the fragment binds said MHC class II molecule.

19. The microparticle of claim 16, wherein the expression product comprises an amino acid sequence essentially identical to a sequence selected from the group consisting of SEQ ID NOS 1-46.

20. The microparticle of claim 14, wherein the expression product comprises a trafficking sequence selected from the group consisting of a sequence which trafficks to endoplasmic reticulum, a sequence which trafficks to a lysosome, a sequence which trafficks to an endosome, a sequence which causes secretion from a cell, and a sequence which trafficks to the nucleus.

21. The microparticle of claim 14, wherein the expression product comprises an amino acid sequence essentially identical to the sequence of an antigenic portion of a tumor antigen.

22. The microparticle of claim 21, wherein the tumor antigen is selected from the group consisting of the antigens listed in Table 3.

23. The microparticle of claim 14, wherein the expression product comprises an amino acid sequence essentially identical to the sequence of an antigenic fragment of a protein naturally expressed by an infectious agent selected from the group consisting of a virus, a bacterium, and a parasitic eukaryote.

24. The microparticle of claim 23, wherein the infectious agent is selected from the group consisting of human papillomavirus, human immunodeficiency virus, herpes simplex virus, hepatitis B virus, hepatitis c virus, *Plasmodium* species, and mycobacteria.

25. A method of administering nucleic acid to an animal, comprising:
providing the microparticle of claim 14; and
introducing the microparticle into the animal.

26. The method of claim 25, wherein the microparticle is provided suspended in a solution.

27. The method of claim 25, wherein the microparticle is injected into the animal.

28. The method of claim 25, wherein the microparticle is implanted into the animal.

29. A microparticle less than about 20 microns in diameter, comprising:
a polymeric matrix; and
a nucleic acid molecule comprising an expression control sequence operatively linked to a coding sequence, wherein the coding sequence encodes a protein which, when expressed, downregulates an immune response in an animal.

30. A process for preparing microparticles, comprising:
(1) providing a first solution comprising a polymer dissolved in an organic solvent;
(2) providing a second solution comprising a nucleic acid dissolved or suspended in a polar or hydrophilic solvent;
(3) mixing the first and second solutions to form a first emulsion; and
(4) mixing the first emulsion with a third solution comprising an organic compound, to form a second emulsion comprising microparticles of polymeric matrix and nucleic acid;
wherein both mixing steps are carried out in a manner that minimizes shearing of the nucleic acid while producing microparticles having a number average smaller than 100 microns in diameter.

31. The process of claim 30, wherein the second solution is prepared by purifying the nucleic acid, then suspending the purified nucleic acid in the polar or hydrophilic solvent.

32. The process of claim 30, wherein the second solution is prepared by precipitating the nucleic acid with alcohol, then suspending the precipitated nucleic acid in the polar or hydrophilic solvent.

33. The process of claim 30, wherein the second solution further comprises an aqueous buffer solution comprising a buffer compound selected from the group consisting of ethylenediaminetetraacetic acid, tris (hydroxymethyl) aminomethane, and combinations thereof.

34. The process of claim 30, wherein the second solution further comprises a stabilizer compound.

35. The process of claim 30, wherein the second solution further comprises a surfactant.

36. The process of claim 30, wherein the second solution further comprises a DNA-condensing agent.

37. The process of claim 30, further comprising resuspending the microparticles in an excipient solution.

38. The process of claim 30, wherein the second emulsion is exposed to an elevated temperature.

39. The process of claim 30, comprising the additional step of mixing the second emulsion with a fourth solution comprising an organic compound.

40. The process of claim 30, comprising the additional step of washing the microparticles with an aqueous solution, thereby producing washed microparticles.

41. The process of claim 40, comprising the additional steps of:
subjecting the washed microparticles to a temperature below 0°C, to produce frozen microparticles; and
lyophilizing the frozen microparticles, to produce lyophilized microparticles.

42. The process of claim 30, comprising the additional step of screening the microparticles to remove essentially all of which are larger than 100 microns in diameter.

43. The process of claim 30, comprising the additional step of screening the microparticles to remove essentially all of which are larger than 20 microns in diameter.

44. A method of administering nucleic acid to an animal, comprising:
providing the microparticle of claim 1; and
introducing the microparticle into the animal.

45. A method of administering nucleic acid to an animal, comprising:
providing the microparticle of claim 2; and
introducing the microparticle into the animal.

46. A method of administering nucleic acid to an animal, comprising:
providing the microparticle of claim 3; and
introducing the microparticle into the animal.

47. A method of administering nucleic acid to an animal, comprising:
providing the microparticle of claim 6; and
introducing the microparticle into the animal.

48. A preparation of microparticles, each of which comprises:
a polymeric matrix;
a proteinaceous antigenic determinant; and
DNA which encodes an antigenic polypeptide.

49. The preparation of claim 48, wherein said antigenic determinant elicits an antibody response in a mammal.

50. The preparation of claim 48, wherein said antigenic polypeptide elicits a T cell response.

51. The preparation of claim 50, wherein said T cell response is a cytotoxic T cell (CTL) response.

52. The preparation of claim 48, wherein said DNA is plasmid DNA.

53. A method of administering nucleic acid to an animal, comprising
providing the preparation of claim 29; and
introducing the preparation into the animal.

54. A method of administering nucleic acid to an animal, comprising
providing the preparation of claim 48; and introducing the preparation into the animal.

55. A preparation of microparticles, each of which comprises a polymeric matrix, nucleic acid, and a stabilizer compound, the polymeric matrix consisting essentially of one or more synthetic polymers having a solubility in water of less than about 1 mg/l, wherein
at least 90% of the microparticles have a diameter less than about 100 microns, and
the nucleic acid is an expression vector selected from the group consisting of RNA molecules, at least 50% of which are closed circles; and circular plasmid DNA molecules.

56. The preparation of claim 55, wherein the stabilizer compound is a cationic compound.

57. The preparation of claim 55, wherein the stabilizer compound is a carbohydrate or a DNA-condensing agent.

58. The preparation of claim 2, wherein the stabilizer compound is a cationic compound.

59. The preparation of claim 2, wherein the stabilizer compound is a carbohydrate or a DNA-condensing agent.

60. The preparation of claim 2 or claim 55, wherein the stabilizer compound is selected from dextrose, sucrose, dextran, polyvinyl alcohol, cyclodextrin, dextran sulfate, cationic peptides or lipids.

61. A method of administering nucleic acid to an animal, comprising providing the preparation of claim 55; and
introducing the preparation into the animal.


SEQUENCE LISTING

(1) GENERAL INFORMATION

(i) APPLICANT: Pangaea, Inc.

(ii) TITLE OF THE INVENTION: MICROPARTICLES FOR DELIVERY
OF NUCLEIC ACID

(iii) NUMBER OF SEQUENCES: 107

(iv) CORRESPONDENCE ADDRESS:
(A) ADDRESSEE: Fish & Richardson, P.C.
(B) STREET: 225 Franklin Street
(C) CITY: Boston
(D) STATE: MA
(E) COUNTRY: US
(F) ZIP: 02110-2804

(v) COMPUTER READABLE FORM:
(A) MEDIUM TYPE: Diskette
(B) COMPUTER: IBM Compatible
(C) OPERATING SYSTEM: Windows95
(D) SOFTWARE: FastSEQ for Windows Version 2.0

(vi) CURRENT APPLICATION DATA:
(A) APPLICATION NUMBER: PCT/US98/----
(B) FILING DATE: 22-JAN-1998

(vii) PRIOR APPLICATION DATA:
(A) APPLICATION NUMBER: 08/787,547
(B) FILING DATE: 22-JAN-1997

(viii) ATTORNEY/AGENT INFORMATION:
(A) NAME: Fraser, Janis K.
(B) REGISTRATION NUMBER: 34,819
(C) REFERENCE/DOCKET NUMBER: 08191/003WO1

(ix) TELECOMMUNICATION INFORMATION:
(A) TELEPHONE: 617-542-5070
(B) TELEFAX: 617-542-8906
(C) TELEX: 200154

(2) INFORMATION FOR SEQ ID NO:1:

(i) SEQUENCE CHARACTERISTICS:
(A) LENGTH: 23 amino acids
(B) TYPE: amino acid
(D) TOPOLOGY: linear

(ii) MOLECULE TYPE: peptide

(xi) SEQUENCE DESCRIPTION: SEQ ID NO:1:

Gly Arg Thr Gln Asp Glu Asn Pro Val Val His Phe Phe Lys Asn Ile
1               5                   10                  15
Val Thr Pro Arg Thr Pro Pro
            20

(2) INFORMATION FOR SEQ ID NO:2:

(i) SEQUENCE CHARACTERISTICS:

(A) LENGTH: 22 amino acids
(B) TYPE: amino acid
(D) TOPOLOGY: linear

(ii) MOLECULE TYPE: peptide

(xi) SEQUENCE DESCRIPTION: SEQ ID NO:2:

Ala Val Tyr Val Tyr Ile Tyr Phe Asn Thr Trp Thr Thr Cys Gln Phe
1               5                   10                  15
Ile Ala Phe Pro Phe Lys
            20

(2) INFORMATION FOR SEQ ID NO:3:

(i) SEQUENCE CHARACTERISTICS:
(A) LENGTH: 13 amino acids
(B) TYPE: amino acid
(D) TOPOLOGY: linear

(ii) MOLECULE TYPE: peptide

(xi) SEQUENCE DESCRIPTION: SEQ ID NO:3:

Phe Lys Met Arg Met Ala Thr Pro Leu Leu Met Gln Ala
1               5                   10

(2) INFORMATION FOR SEQ ID NO:4:

(i) SEQUENCE CHARACTERISTICS:
(A) LENGTH: 36 amino acids
(B) TYPE: amino acid
(D) TOPOLOGY: linear

(ii) MOLECULE TYPE: peptide

(xi) SEQUENCE DESCRIPTION: SEQ ID NO:4:

Thr Val Gly Leu Gln Leu Ile Gln Leu Ile Asn Val Asp Glu Val Asn
1               5                   10                  15
Gln Ile Val Thr Thr Asn Val Arg Leu Lys Gln Gln Trp Val Asp Tyr
            20                  25                  30
Asn Leu Lys Trp
        35

(2) INFORMATION FOR SEQ ID NO:5:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:5:

Gln Ile Val Thr Thr Asn Val Arg Leu Lys Gln Gln Trp Val Asp Tyr
1               5                   10                  15
Asn Leu Lys Trp
            20

(2) INFORMATION FOR SEQ ID NO:6:

(i) SEQUENCE CHARACTERISTICS:
(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:6:

Gln Trp Val Asp Tyr Asn Leu
1               5

(2) INFORMATION FOR SEQ ID NO:7:

(i) SEQUENCE CHARACTERISTICS:
(A) LENGTH: 18 amino acids
(B) TYPE: amino acid
(D) TOPOLOGY: linear

(ii) MOLECULE TYPE: peptide

(xi) SEQUENCE DESCRIPTION: SEQ ID NO:7:

Gly Gly Val Lys Lys Ile His Ile Pro Ser Glu Lys Ile Trp Arg Pro
1               5                   10                  15
Asp Leu

(2) INFORMATION FOR SEQ ID NO:8:

(i) SEQUENCE CHARACTERISTICS:
(A) LENGTH: 12 amino acids
(B) TYPE: amino acid
(D) TOPOLOGY: linear

(ii) MOLECULE TYPE: peptide

(xi) SEQUENCE DESCRIPTION: SEQ ID NO:8:

Ala Ile Val Lys Phe Thr Lys Val Leu Leu Gln Tyr
1               5                   10

(2) INFORMATION FOR SEQ ID NO:9:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:9:

Trp Thr Pro Pro Ala Ile Phe Lys Ser Tyr Cys Glu Ile Ile Val Thr
1               5                   10                  15
His Phe Pro Phe

            20

(2) INFORMATION FOR SEQ ID NO:10:

(i) SEQUENCE CHARACTERISTICS:
(A) LENGTH: 13 amino acids
(B) TYPE: amino acid
(D) TOPOLOGY: linear

(ii) MOLECULE TYPE: peptide

(xi) SEQUENCE DESCRIPTION: SEQ ID NO:10:

Met Lys Leu Gly Thr Trp Thr Tyr Asp Gly Ser Val Val
1               5                   10

(2) INFORMATION FOR SEQ ID NO:11:

(i) SEQUENCE CHARACTERISTICS:
(A) LENGTH: 13 amino acids
(B) TYPE: amino acid
(D) TOPOLOGY: linear

(ii) MOLECULE TYPE: peptide

(xi) SEQUENCE DESCRIPTION: SEQ ID NO:11:

Met Lys Leu Gly Ile Trp Thr Tyr Asp Gly Ser Val Val
1               5                   10

(2) INFORMATION FOR SEQ ID NO:12:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:12:

Trp Thr Tyr Asp Gly Ser Val Val Ala
1               5

(2) INFORMATION FOR SEQ ID NO:13:

(i) SEQUENCE CHARACTERISTICS:
(A) LENGTH: 17 amino acids
(B) TYPE: amino acid
(D) TOPOLOGY: linear

(ii) MOLECULE TYPE: peptide

(xi) SEQUENCE DESCRIPTION: SEQ ID NO:13:

Ser Cys Cys Pro Asp Thr Pro Tyr Leu Asp Ile Thr Tyr His Phe Val
1               5                   10                  15
Met

(2) INFORMATION FOR SEQ ID NO:14:

(i) SEQUENCE CHARACTERISTICS:
(A) LENGTH: 18 amino acids
(B) TYPE: amino acid
(D) TOPOLOGY: linear

(ii) MOLECULE TYPE: peptide

(xi) SEQUENCE DESCRIPTION: SEQ ID NO:14:

Asp Thr Pro Tyr Leu Asp Ile Thr Tyr His Phe Val Met Gln Arg Leu
1               5                   10                  15
Pro Leu

(2) INFORMATION FOR SEQ ID NO:15:

(i) SEQUENCE CHARACTERISTICS:
(A) LENGTH: 21 amino acids
(B) TYPE: amino acid
(D) TOPOLOGY: linear

(ii) MOLECULE TYPE: peptide

(xi) SEQUENCE DESCRIPTION: SEQ ID NO:15:

Phe Ile Val Asn Val Ile Ile Pro Cys Leu Leu Phe Ser Phe Leu Thr
1               5                   10                  15
Gly Leu Val Phe Tyr
            20

(2) INFORMATION FOR SEQ ID NO:16:

(i) SEQUENCE CHARACTERISTICS:
(A) LENGTH: 13 amino acids
(B) TYPE: amino acid
(D) TOPOLOGY: linear

(ii) MOLECULE TYPE: peptide

(xi) SEQUENCE DESCRIPTION: SEQ ID NO:16:

Leu Leu Val Ile Val Glu Leu Ile Pro Ser Thr Ser Ser
1               5                   10

(2) INFORMATION FOR SEQ ID NO:17:

(i) SEQUENCE CHARACTERISTICS:
(A) LENGTH: 19 amino acids
(B) TYPE: amino acid
(D) TOPOLOGY: linear

(ii) MOLECULE TYPE: peptide

(xi) SEQUENCE DESCRIPTION: SEQ ID NO:17:

Ser Thr His Val Met Pro Asn Trp Val Arg Lys Val Phe Ile Asp Thr
1               5                   10                  15
Ile Pro Asn

(2) INFORMATION FOR SEQ ID NO:18:

(i) SEQUENCE CHARACTERISTICS:
(A) LENGTH: 18 amino acids
(B) TYPE: amino acid
(D) TOPOLOGY: linear

(ii) MOLECULE TYPE: peptide

(xi) SEQUENCE DESCRIPTION: SEQ ID NO:18:

Asn Trp Val Arg Lys Val Phe Ile Asp Thr Ile Pro Asn Ile Met Phe
1               5                   10                  15
Phe Ser

(2) INFORMATION FOR SEQ ID NO:19:

(i) SEQUENCE CHARACTERISTICS:
(A) LENGTH: 18 amino acids
(B) TYPE: amino acid
(D) TOPOLOGY: linear

(ii) MOLECULE TYPE: peptide

(xi) SEQUENCE DESCRIPTION: SEQ ID NO:19:

Ile Pro Asn Ile Met Phe Phe Ser Thr Met Lys Arg Pro Ser Arg Glu
1               5                   10                  15
Lys Gln

(2) INFORMATION FOR SEQ ID NO:20:

(i) SEQUENCE CHARACTERISTICS:
(A) LENGTH: 16 amino acids
(B) TYPE: amino acid
(D) TOPOLOGY: linear

(ii) MOLECULE TYPE: peptide

(xi) SEQUENCE DESCRIPTION: SEQ ID NO:20:

Ala Ala Ala Glu Trp Lys Tyr Val Ala Met Val Met Asp His Ile Leu
1               5                   10                  15

(2) INFORMATION FOR SEQ ID NO:21:

(i) SEQUENCE CHARACTERISTICS:
(A) LENGTH: 19 amino acids
(B) TYPE: amino acid
(D) TOPOLOGY: linear

(ii) MOLECULE TYPE: peptide

(xi) SEQUENCE DESCRIPTION: SEQ ID NO:21:

Ile Ile Gly Thr Leu Ala Val Phe Ala Gly Arg Leu Ile Glu Leu Asn

1               5                   10                  15
Gln Gln Gly

(2) INFORMATION FOR SEQ ID NO:22:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:22:

Gly Gln Thr Ile Glu Trp Ile Phe Ile Asp Pro Glu Ala Phe Thr Glu
1               5                   10                  15
Asn Gly Glu Trp
            20

(2) INFORMATION FOR SEQ ID NO:23:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:23:

Met Ala His Tyr Asn Arg Val Pro Ala Leu Pro Phe Pro Gly Asp Pro
1               5                   10                  15
Arg Pro Tyr Leu
            20

(2) INFORMATION FOR SEQ ID NO:24:

(i) SEQUENCE CHARACTERISTICS:
(A) LENGTH: 15 amino acids
(B) TYPE: amino acid
(D) TOPOLOGY: linear

(ii) MOLECULE TYPE: peptide

(xi) SEQUENCE DESCRIPTION: SEQ ID NO:24:

Leu Asn Ser Lys Ile Ala Phe Lys Ile Val Ser Gln Glu Pro Ala
1               5                   10                  15

(2) INFORMATION FOR SEQ ID NO:25:

(i) SEQUENCE CHARACTERISTICS:
(A) LENGTH: 15 amino acids
(B) TYPE: amino acid
(D) TOPOLOGY: linear

(ii) MOLECULE TYPE: peptide

(xi) SEQUENCE DESCRIPTION: SEQ ID NO:25:

Thr Pro Met Phe Leu Leu Ser Arg Asn Thr Gly Glu Val Arg Thr
1               5                   10                  15

(2) INFORMATION FOR SEQ ID NO:26:

(i) SEQUENCE CHARACTERISTICS:
(A) LENGTH: 16 amino acids
(B) TYPE: amino acid
(D) TOPOLOGY: linear

(ii) MOLECULE TYPE: peptide

(xi) SEQUENCE DESCRIPTION: SEQ ID NO:26:

Pro Leu Gly Phe Phe Pro Asp His Gln Leu Asp Pro Ala Phe Gly Ala
1               5                   10                  15

(2) INFORMATION FOR SEQ ID NO:27:

(i) SEQUENCE CHARACTERISTICS:
(A) LENGTH: 17 amino acids
(B) TYPE: amino acid
(D) TOPOLOGY: linear

(ii) MOLECULE TYPE: peptide

(xi) SEQUENCE DESCRIPTION: SEQ ID NO:27:

Leu Gly Phe Phe Pro Asp His Gln Leu Asp Pro Ala Phe Gly Ala Asn
1               5                   10                  15
Ser

(2) INFORMATION FOR SEQ ID NO:28:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:28:

Phe Phe Leu Leu Thr Arg Ile Leu Thr Ile
1               5                   10

(2) INFORMATION FOR SEQ ID NO:29:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:29:

Arg Ile Leu Thr Ile Pro Gln Ser Leu Asp
1               5                   10

(2) INFORMATION FOR SEQ ID NO:30:

(i) SEQUENCE CHARACTERISTICS:
(A) LENGTH: 13 amino acids
(B) TYPE: amino acid
(D) TOPOLOGY: linear

(ii) MOLECULE TYPE: peptide

(xi) SEQUENCE DESCRIPTION: SEQ ID NO:30:

Thr Pro Thr Leu Val Glu Val Ser Arg Asn Leu Gly Lys
1               5                   10

(2) INFORMATION FOR SEQ ID NO:31:

(i) SEQUENCE CHARACTERISTICS:
(A) LENGTH: 12 amino acids
(B) TYPE: amino acid
(D) TOPOLOGY: linear

(ii) MOLECULE TYPE: peptide

(xi) SEQUENCE DESCRIPTION: SEQ ID NO:31:

Ala Lys Thr Ile Ala Tyr Asp Glu Glu Ala Arg Arg
1               5                   10

(2) INFORMATION FOR SEQ ID NO:32:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:32:

Val Val Thr Val Arg Ala Glu Arg Pro Gly
1               5                   10

(2) INFORMATION FOR SEQ ID NO:33:

(i) SEQUENCE CHARACTERISTICS:
(A) LENGTH: 21 amino acids
(B) TYPE: amino acid
(D) TOPOLOGY: linear

(ii) MOLECULE TYPE: peptide

(xi) SEQUENCE DESCRIPTION: SEQ ID NO:33:

Ser Gln Arg His Gly Ser Lys Tyr Leu Ala Thr Ala Ser Thr Met Asp
1               5                   10                  15
His Ala Arg His Gly
            20

(2) INFORMATION FOR SEQ ID NO:34:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:34:

Arg Asp Thr Gly Ile Leu Asp Ser Ile Gly Arg Phe Phe Gly Gly Asp
1               5                   10                  15
Arg Gly Ala Pro
            20

(2) INFORMATION FOR SEQ ID NO:35:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:35:

Gln Lys Ser His Gly Arg Thr Gln Asp Glu Asn Pro Val Val His Phe
1               5                   10                  15
Phe Lys Asn Ile
            20

(2) INFORMATION FOR SEQ ID NO:36:

(i) SEQUENCE CHARACTERISTICS:
(A) LENGTH: 14 amino acids
(B) TYPE: amino acid
(D) TOPOLOGY: linear

(ii) MOLECULE TYPE: peptide

(xi) SEQUENCE DESCRIPTION: SEQ ID NO:36:

Asp Glu Asn Pro Val Val His Phe Phe Lys Asn Ile Val Thr
1               5                   10

(2) INFORMATION FOR SEQ ID NO:37:

(i) SEQUENCE CHARACTERISTICS:
(A) LENGTH: 15 amino acids
(B) TYPE: amino acid
(D) TOPOLOGY: linear

(ii) MOLECULE TYPE: peptide

(xi) SEQUENCE DESCRIPTION: SEQ ID NO:37:

Glu Asn Pro Val Val His Phe Phe Lys Asn Ile Val Thr Pro Arg
1               5                   10                  15

(2) INFORMATION FOR SEQ ID NO:38:

(i) SEQUENCE CHARACTERISTICS:
(A) LENGTH: 13 amino acids
(B) TYPE: amino acid
(D) TOPOLOGY: linear

(ii) MOLECULE TYPE: peptide

(xi) SEQUENCE DESCRIPTION: SEQ ID NO:38:

His Phe Phe Lys Asn Ile Val Thr Pro Arg Thr Pro Pro
1               5                   10

(2) INFORMATION FOR SEQ ID NO:39:

(i) SEQUENCE CHARACTERISTICS:
(A) LENGTH: 14 amino acids
(B) TYPE: amino acid
(D) TOPOLOGY: linear

(ii) MOLECULE TYPE: peptide

(xi) SEQUENCE DESCRIPTION: SEQ ID NO:39:

Lys Gly Phe Lys Gly Val Asp Ala Gln Gly Thr Leu Ser Lys
1               5                   10

(2) INFORMATION FOR SEQ ID NO:40:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:40:

Val Asp Ala Gln Gly Thr Leu Ser Lys Ile Phe Lys Leu Gly Gly Arg
1               5                   10                  15
Asp Ser Arg Ser
            20

(2) INFORMATION FOR SEQ ID NO:41:

(i) SEQUENCE CHARACTERISTICS:
(A) LENGTH: 19 amino acids
(B) TYPE: amino acid
(D) TOPOLOGY: linear

(ii) MOLECULE TYPE: peptide

(xi) SEQUENCE DESCRIPTION: SEQ ID NO:41:

Leu Met Gln Tyr Ile Asp Ala Asn Ser Lys Phe Ile Gly Ile Thr Glu
1               5                   10                  15
Leu Lys Lys

(2) INFORMATION FOR SEQ ID NO:42:

(i) SEQUENCE CHARACTERISTICS:
(A) LENGTH: 13 amino acids
(B) TYPE: amino acid
(D) TOPOLOGY: linear

(ii) MOLECULE TYPE: peptide

(xi) SEQUENCE DESCRIPTION: SEQ ID NO:42:

Gln Tyr Ile Lys Ala Asn Ser Lys Phe Ile Gly Ile Thr
1               5                   10

(2) INFORMATION FOR SEQ ID NO:43:

(i) SEQUENCE CHARACTERISTICS:
(A) LENGTH: 14 amino acids
(B) TYPE: amino acid
(D) TOPOLOGY: linear

(ii) MOLECULE TYPE: peptide

(xi) SEQUENCE DESCRIPTION: SEQ ID NO:43:

Phe Asn Asn Phe Thr Val Ser Phe Trp Leu Arg Val Pro Lys
1               5                   10

(2) INFORMATION FOR SEQ ID NO:44:

(i) SEQUENCE CHARACTERISTICS:
(A) LENGTH: 15 amino acids
(B) TYPE: amino acid
(D) TOPOLOGY: linear

(ii) MOLECULE TYPE: peptide

(xi) SEQUENCE DESCRIPTION: SEQ ID NO:44:

Ser Phe Trp Leu Arg Val Pro Lys Val Ser Ala Ser His Leu Glu
1               5                   10                  15

(2) INFORMATION FOR SEQ ID NO:45:

(i) SEQUENCE CHARACTERISTICS:
(A) LENGTH: 16 amino acids
(B) TYPE: amino acid
(D) TOPOLOGY: linear

(ii) MOLECULE TYPE: peptide

(xi) SEQUENCE DESCRIPTION: SEQ ID NO:45:

Lys Phe Ile Ile Lys Arg Tyr Thr Pro Asn Asn Glu Ile Asp Ser Phe
1               5                   10                  15

(2) INFORMATION FOR SEQ ID NO:46:

(i) SEQUENCE CHARACTERISTICS:
(A) LENGTH: 12 amino acids
(B) TYPE: amino acid
(D) TOPOLOGY: linear

(ii) MOLECULE TYPE: peptide

(xi) SEQUENCE DESCRIPTION: SEQ ID NO:46:

Gly Gln Ile Gly Asn Asp Pro Asn Arg Asp Ile Leu
1               5                   10

(2) INFORMATION FOR SEQ ID NO:47:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:47:

Ala Ala Arg Ala Val Phe Leu Ala Leu
1               5

(2) INFORMATION FOR SEQ ID NO:48:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:48:

Tyr Arg Pro Arg Pro Arg Arg Tyr
1               5

(2) INFORMATION FOR SEQ ID NO:49:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:49:

Glu Ala Asp Pro Thr Gly His Ser Tyr
1               5

(2) INFORMATION FOR SEQ ID NO:50:

(i) SEQUENCE CHARACTERISTICS:
(A) LENGTH: 9 amino acids
(B) TYPE: amino acid

(D) TOPOLOGY: linear

(ii) MOLECULE TYPE: peptide

(xi) SEQUENCE DESCRIPTION: SEQ ID NO:50:

Ser Ala Tyr Gly Glu Pro Arg Lys Leu
1               5

(2) INFORMATION FOR SEQ ID NO:51:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:51:

Glu Val Asp Pro Ile Gly His Leu Tyr
1               5

(2) INFORMATION FOR SEQ ID NO:52:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:52:

Phe Leu Trp Gly Pro Arg Ala Leu Val
1               5

(2) INFORMATION FOR SEQ ID NO:53:

(i) SEQUENCE CHARACTERISTICS:
(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:53:

Gly Ile Gly Ile Leu Thr Val
1               5

(2) INFORMATION FOR SEQ ID NO:54:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:54:

Ile Leu Thr Val Ile Leu Gly Val
1               5

(2) INFORMATION FOR SEQ ID NO:55:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:55:

Ser Thr Ala Pro Pro Ala His Gly Val
1               5

(2) INFORMATION FOR SEQ ID NO:56:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:56:

Glu Glu Lys Leu Ile Val Val Leu Phe
1               5

(2) INFORMATION FOR SEQ ID NO:57:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:57:

Met Leu Leu Ala Val Leu Tyr Cys Leu
1               5

(2) INFORMATION FOR SEQ ID NO:58:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:58:

Ser Glu Ile Trp Arg Asp Ile Asp Phe
1               5

(2) INFORMATION FOR SEQ ID NO:59:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:59:

Ala Phe Leu Pro Trp His Arg Leu Phe
1               5

(2) INFORMATION FOR SEQ ID NO:60:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:60:

Tyr Met Asn Gly Thr Met Ser Gln Val
1               5

(2) INFORMATION FOR SEQ ID NO:61:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:61:

Lys Thr Trp Gly Gln Tyr Trp Gln Val
1               5

(2) INFORMATION FOR SEQ ID NO:62:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:62:

Ile Thr Asp Gln Val Pro Phe Ser Val
1               5

(2) INFORMATION FOR SEQ ID NO:63:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:63:

Tyr Leu Glu Pro Gly Pro Thr Val Ala
1               5

(2) INFORMATION FOR SEQ ID NO:64:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:64:

Leu Leu Asp Gly Thr Ala Thr Leu Arg Leu
1               5                   10

(2) INFORMATION FOR SEQ ID NO:65:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:65:

Glu Leu Asn Glu Ala Leu Glu Leu Glu Lys
1               5                   10

(2) INFORMATION FOR SEQ ID NO:66:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:66:

Ser Thr Pro Pro Pro Gly Thr Arg Val
1               5

(2) INFORMATION FOR SEQ ID NO:67:

(i) SEQUENCE CHARACTERISTICS:
(A) LENGTH: 11 amino acids
(B) TYPE: amino acid

(D) TOPOLOGY: linear

(ii) MOLECULE TYPE: peptide

(xi) SEQUENCE DESCRIPTION: SEQ ID NO:67:

Leu Leu Pro Glu Asn Asn Val Leu Ser Pro Leu
1               5                   10

(2) INFORMATION FOR SEQ ID NO:68:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:68:

Leu Leu Gly Arg Asn Ser Phe Glu Val
1               5

(2) INFORMATION FOR SEQ ID NO:69:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:69:

Arg Met Pro Glu Ala Ala Pro Pro Val
1               5

(2) INFORMATION FOR SEQ ID NO:70:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:70:

Lys Ile Phe Gly Ser Leu Ala Phe Leu
1               5

(2) INFORMATION FOR SEQ ID NO:71:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:71:

Ile Ile Ser Ala Val Val Gly Ile Leu
1               5

(2) INFORMATION FOR SEQ ID NO:72:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:72:

Cys Leu Thr Ser Thr Val Gln Leu Val
1               5

(2) INFORMATION FOR SEQ ID NO:73:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:73:

Tyr Leu Glu Asp Val Arg Leu Val
1               5

(2) INFORMATION FOR SEQ ID NO:74:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:74:

Val Leu Val Lys Ser Pro Asn His Val
1               5

(2) INFORMATION FOR SEQ ID NO:75:

(i) SEQUENCE CHARACTERISTICS:
(A) LENGTH: 12 amino acids
(B) TYPE: amino acid
(D) TOPOLOGY: linear

(ii) MOLECULE TYPE: peptide

(xi) SEQUENCE DESCRIPTION: SEQ ID NO:75:

Arg Phe Arg Glu Leu Val Ser Glu Phe Ser Arg Met

1                    5                    10

(2) INFORMATION FOR SEQ ID NO:76:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:76:

Leu Leu Arg Leu Ser Glu Pro Ala Glu Leu
1               5                   10

(2) INFORMATION FOR SEQ ID NO:77:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:77:

Asp Leu Pro Thr Gln Glu Pro Ala Leu
1               5

(2) INFORMATION FOR SEQ ID NO:78:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:78:

Lys Leu Gln Cys Val Asp Leu His Val
1               5

(2) INFORMATION FOR SEQ ID NO:79:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:79:

Val Leu Val Ala Ser Arg Gly Arg Ala Val
1               5                   10

(2) INFORMATION FOR SEQ ID NO:80:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:80:

Val Leu Val His Pro Gln Trp Val Leu
1               5

(2) INFORMATION FOR SEQ ID NO:81:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:81:

Asp Met Ser Leu Leu Lys Asn Arg Phe Leu
1               5                   10

(2) INFORMATION FOR SEQ ID NO:82:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:82:

Gln Trp Asn Ser Thr Ala Phe His Gln
1               5

(2) INFORMATION FOR SEQ ID NO:83:

(i) SEQUENCE CHARACTERISTICS:
(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:83:

Val Leu Gln Ala Gly Phe Phe
1               5

(2) INFORMATION FOR SEQ ID NO:84:

(i) SEQUENCE CHARACTERISTICS:
(A) LENGTH: 8 amino acids

(B) TYPE: amino acid
(D) TOPOLOGY: linear

(ii) MOLECULE TYPE: peptide

(xi) SEQUENCE DESCRIPTION: SEQ ID NO:84:

Leu Leu Leu Cys Leu Ile Phe Leu
1               5

(2) INFORMATION FOR SEQ ID NO:85:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:85:

Leu Leu Asp Tyr Gln Gly Met Leu
1               5

(2) INFORMATION FOR SEQ ID NO:86:

(i) SEQUENCE CHARACTERISTICS:
(A) LENGTH: 6 amino acids
(B) TYPE: amino acid
(D) TOPOLOGY: linear

(ii) MOLECULE TYPE: peptide

(xi) SEQUENCE DESCRIPTION: SEQ ID NO:86:

Leu Leu Val Pro Phe Val
1               5

(2) INFORMATION FOR SEQ ID NO:87:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:87:

Ser Ile Leu Ser Pro Phe Met Pro Leu Leu
1               5                   10

(2) INFORMATION FOR SEQ ID NO:88:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:88:

Pro Leu Leu Pro Ile Phe Phe Cys Leu
1               5

(2) INFORMATION FOR SEQ ID NO:89:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:89:

Ile Leu Ser Thr Leu Pro Glu Thr Thr Val
1               5                   10

(2) INFORMATION FOR SEQ ID NO:90:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:90:

Phe Leu Pro Ser Asp Phe Phe Pro Ser Val
1               5                   10

(2) INFORMATION FOR SEQ ID NO:91:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:91:

Lys Leu His Leu Tyr Ser His Pro Ile
1               5

(2) INFORMATION FOR SEQ ID NO:92:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:92:

Ala Leu Met Pro Leu Tyr Ala Cys Ile
1               5

(2) INFORMATION FOR SEQ ID NO:93:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:93:

His Leu Tyr Ser His Pro Ile Ile Leu
1               5

(2) INFORMATION FOR SEQ ID NO:94:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:94:

Phe Leu Leu Ser Leu Gly Ile His Leu
1               5

(2) INFORMATION FOR SEQ ID NO:95:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:95:

His Leu Leu Val Gly Ser Ser Gly Leu
1               5

(2) INFORMATION FOR SEQ ID NO:96:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:96:

Gly Leu Ser Arg Tyr Val Ala Arg Leu
1               5

(2) INFORMATION FOR SEQ ID NO:97:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:97:

Leu Leu Ala Gln Phe Thr Ser Ala Ile
1               5

(2) INFORMATION FOR SEQ ID NO:98:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:98:

Tyr Met Asp Asp Val Val Leu Gly Ala
1               5

(2) INFORMATION FOR SEQ ID NO:99:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:99:

Gly Leu Tyr Ser Ser Thr Val Pro Val
1               5

(2) INFORMATION FOR SEQ ID NO:100:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:100:

Asn Leu Ser Trp Leu Ser Leu Asp Val
1               5

(2) INFORMATION FOR SEQ ID NO:101:

(i) SEQUENCE CHARACTERISTICS:

(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:101:

Lys Leu Pro Gln Leu Cys Thr Glu Leu
1               5

(2) INFORMATION FOR SEQ ID NO:102:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:102:

Leu Gln Thr Thr Ile His Asp Ile Ile
1               5

(2) INFORMATION FOR SEQ ID NO:103:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:103:

Phe Ala Phe Arg Asp Leu Cys Ile Val
1               5

(2) INFORMATION FOR SEQ ID NO:104:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:104:

Tyr Met Leu Asp Leu Gln Pro Glu Thr
1               5

(2) INFORMATION FOR SEQ ID NO:105:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:105:

Thr Leu His Glu Tyr Met Leu Asp Leu
1               5

(2) INFORMATION FOR SEQ ID NO:106:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:106:

Leu Leu Met Gly Thr Leu Gly Ile Val
1               5

(2) INFORMATION FOR SEQ ID NO:107:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:107:

Thr Leu Gly Ile Val Cys Pro Ile
1               5